# EUROPEAN PATENT SPECIFICATION

(11) **EP 4 179 724 B1**
(45) Date of publication and mention of the grant of the patent: **25.11.2026**
(21) Application number: 21749921.9
(22) Date of filing: 07.07.2021
(51) Int. Cl.: A61B 1/06, H04N 23/74, H04N 23/73, H04N 23/72, H04N 23/71

(54) **APPARATUSES, SYSTEMS, AND METHODS FOR DISCOUNTING AN OBJECT WHILE MANAGING AUTO-EXPOSURE OF IMAGE FRAMES DEPICTING THE OBJECT**
VORRICHTUNGEN, SYSTEME UND VERFAHREN ZUM RABATTIEREN EINES OBJEKTS WÄHREND DER VERWALTUNG DER AUTOMATISCHEN BELICHTUNG VON DAS OBJEKT DARSTELLENDEN EINZELBILDERN
APPAREILS, SYSTÈMES ET PROCÉDÉS POUR DÉCOMPTER UN OBJET TOUT EN GÉRANT L'AUTO-EXPOSITION DE TRAMES D'IMAGE REPRÉSENTANT L'OBJET

(30) Priority: 10.07.2020 US 202063050598 P
(43) Date of publication of application: 17.05.2023
(73) Proprietor: Intuitive Surgical Operations, Inc., Sunnyvale, CA 94086 (US)
(72) Inventor: HE, Zhen, Sunnyvale, California 94086 (US); DICARLO, Jeffrey M., Sunnyvale, California 94086 (US)
(74) Representative: MacDougall, Alan John Shaw
(86) International application number: PCT/US2021/040712
(87) International publication number: WO 2022/011029

(56) References cited:
- US-A1- 2005 057 666
- US-A1- 2014 046 341

## Description

### BACKGROUND INFORMATION

Auto-exposure algorithms operate by analyzing image frames to determine how much light is present at a scene depicted by the image frames and by updating, based on this analysis, auto-exposure parameters of an image capture device capturing the image frames. In this manner, the auto-exposure parameters may be continuously updated to cause the image capture device to provide a desired amount of exposure for image frames being captured. Without good auto-exposure management, detail may be lost during the image capture process by either over-exposure (e.g., where details are lost because of saturation and the image looks too bright) or under-exposure (e.g., where details are lost because of noise and the image looks too dark).

While conventional auto-exposure algorithms adequately serve many types of images, challenges may be presented by images depicting an object that has a different luminance than other depicted objects in the images, particularly when the object takes up a relatively large portion of the image and the object is not likely to be of particular interest to a viewer of the image. When conventional auto-exposure algorithms process image frames that depict such objects, the algorithms are likely to overexpose or underexpose the image frames and/or to encounter other undesirable issues (e.g., brightness inconsistency as the object moves in and out of frame, etc.).

US 2014/046341 A1 relates to auto exposure control for a camera in a robotic surgical system. A region of interest is determined based on known locations of surgical instrument end effectors, and light level statistics from that region are used to adjust the camera exposure.

US 2005/057666 A1 relates to region based automatic exposure and gain control. A region of interest is defined, image tiles are assigned weights, and exposure and gain values are computed based on weighted pixel data from the region of interest.

The present invention provides an apparatus and method as set out in the appended independent claims. Optional features are defined in the dependent claims.

### BRIEF DESCRIPTION OF THE DRAWINGS

The accompanying drawings illustrate various embodiments and are a part of the specification. The illustrated embodiments are merely examples and do not limit the scope of the disclosure. Throughout the drawings, identical or similar reference numbers designate identical or similar elements.
FIG. 1 shows an illustrative auto-exposure management apparatus for managing auto-exposure of image frames according to principles described herein.
FIG. 2 shows an illustrative auto-exposure management method for managing auto-exposure of image frames according to principles described herein.
FIG. 3 shows an illustrative auto-exposure management system for managing auto-exposure of image frames according to principles described herein.
FIG. 4A illustrates how local characteristics may be employed to identify an object region corresponding to a depiction of an illustrative object portrayed in an illustrative image frame according to principles described herein.
FIG. 4B illustrates how global characteristics may be employed to identify an object region corresponding to a depiction of an illustrative object portrayed in an illustrative image frame according to principles described herein.
FIG. 4C illustrates how pixel units associated with an identified object region may be discounted as part of the determination of frame auto-exposure data points for an illustrative image frame according to principles described herein.
FIG. 5 shows an illustrative flow diagram for managing auto-exposure of image frames according to principles described herein.
FIG. 6 shows an illustrative flow diagram for identifying an object region within an illustrative image frame according to principles described herein.
FIGS. 7A-7B show illustrative geometric characteristics that may be analyzed within a color space to facilitate object region identification within an illustrative image frame according to principles described herein.
FIG. 8 shows an illustrative range of weight values that may be assigned to a pixel unit to indicate a confidence level that the pixel unit corresponds to a depiction of an object portrayed in an image frame according to principles described herein.
FIG. 9 shows an illustrative flow diagram for determining a frame auto-exposure value and a frame auto-exposure target based on weighted pixel units according to principles described herein.
FIG. 10 shows an illustrative technique for updating an auto-exposure parameter according to principles described herein.
FIG. 11 shows an illustrative computer-assisted medical system according to principles described herein.
FIG. 12 shows an illustrative computing system according to principles described herein.

### DETAILED DESCRIPTION

Apparatuses, systems, and methods for managing auto-exposure of image frames are described herein. As mentioned above, auto-exposure management of image frames depicting certain types of objects may be associated with unique challenges. For example, if one particular object has a different luminance than other content depicted in an image frame (e.g., if the object is significantly darker or brighter than other content), the object may significantly affect an average auto-exposure value or auto-exposure target for the image frame (e.g., pulling the average up or down to a significant extent). The larger the object is relative to the image frame, the more pronounced this effect may be. If the object is a subjectively important part of what the image frame depicts (e.g., something that a viewer of the image frame is likely to want to see in detail), it may be desirable for the object to influence the auto-exposure management in this way and a conventional auto-exposure algorithms may perform adequately. However, if the object is an extraneous object that is necessarily depicted in the scene but is unlikely to be something the viewer desires to focus on or view in detail relative to other content (e.g., a foreign object distinct from environmental imagery of the scene), the object's effect on the average auto-exposure value and/or target of the image frame may be undesirable. For example, as auto-exposure management attempts to provide suitable exposure for the object, the exposure of other content depicted in the image frame (e.g., environmental imagery of the scene that may be more desirable for a user to view in detail) may be compromised. Specifically, other objects and/or scene content depicted in the image frame may become at least somewhat overexposed or underexposed due to the undesirable influence of the extraneous object on the auto-exposure properties of the image frame.

As one example of where this type of issue may come into play, an endoscopic image capture device capturing an internal view of a body during a medical procedure on the body (e.g., a surgical procedure, etc.) will be considered. In this situation, a viewer of the endoscopic imagery (e.g., a surgeon or other person assisting with the medical procedure) may desire to see detail of anatomy (e.g., tissue) present in the internal view. However, one or more other objects that are necessarily present at the scene may fit one or more of the following criteria for extraneous objects: (1) being significantly different in appearance than other imagery of the scene (e.g., being significantly darker or brighter than environmental imagery of the scene), or (2) being unlikely to be an important area of focus for a viewer of the image. For example, image frames captured by an endoscope of a computer-assisted medical system (e.g., a single-port computer-assisted medical system) may depict, together with imagery of internal anatomy of the body, one or more extraneous objects used to accomplish the medical procedure. One example of an extraneous object may be a dark-colored shaft of an instrument being used to manipulate the tissue as part of the medical procedure. For instance, a shaft of an instrument may be covered by a dark sheath and may be visible in the image frame as the instrument is used to perform tissue manipulation operations during the procedure. Similarly, an ultrasound probe, a head of an instrument, a tool carried by the instrument, and/or other instrument-related objects may fit the criteria of extraneous objects that are likely to undesirably influence the auto-exposure management in an endoscopic scene. Another example of an extraneous object may be brightly-colored (e.g., white) gauze or other such material (e.g., mesh material for treating a hernia, etc.) that is used as part of the medical procedure.

A viewer of image frames captured in this scenario (e.g., medical staff members assisting in performing the medical procedure) may desire to view detail of anatomical content (e.g., body tissue, blood, etc.) rather than detail of large and/or dark instrument shafts or other such extraneous objects likely to be present in captured images. As such, auto-exposure management apparatuses, systems, and methods described herein may perform operations to identify regions of an image frame that are likely to correspond to a depiction of (e.g., depict or make up part of a depiction of) an extraneous object such as an instrument shaft so that these object regions can be discounted (e.g., ignored or downplayed) as factors on which the auto-exposure management is based. In this way, the extraneous objects may be less likely to undesirably pull the average luminance of the scene up or down, thereby mitigating or resolving issues described above related to undesired overexposure or underexposure of tissue content. Moreover, auto-exposure management described herein may also help stabilize the auto-exposure properties (e.g., average luminance, etc.) of image frame sequences that could otherwise vary widely as instruments and other extraneous objects go in and out of frame, thereby causing flicker and inconsistent auto-exposure of the scene. As such, auto-exposure management described herein may also mitigate or resolve brightness fluctuation issues caused by moving instruments and other related issues (e.g., distraction to viewers, eye fatigue induced in viewers, etc.).

Medical procedure examples involving endoscopic views in which instruments and other extraneous objects are depicted alongside body tissue and anatomy will be referred to throughout this description to illustrate various aspects of the claimed subject matter. However, it will be understood that such endoscopic images are only intended as examples, and the principles described herein may be applied, in various implementations, to any suitable types of content as may serve a particular application or use case. As a few additional examples, for instance, auto-exposure management described herein may find application in photographic applications in which an object is likely to be present in the scene such as a dark- or light-colored tripod holding the camera, a boom microphone, a misplaced thumb or finger blocking part of the lens of a camera, and/or other such extraneous objects likely to undesirably influence the auto-exposure management of an image frame sequence.

Various specific embodiments will now be described in detail with reference to the figures. It will be understood that the specific embodiments described below are provided as non-limiting examples of how various novel and inventive principles may be applied in various situations. Additionally, it will be understood that other examples not explicitly described herein may also be captured by the scope of the claims set forth below. Auto-exposure management apparatuses, systems, and methods described herein may provide any of the benefits mentioned above, as well as various additional and/or alternative benefits that will be described and/or made apparent below.

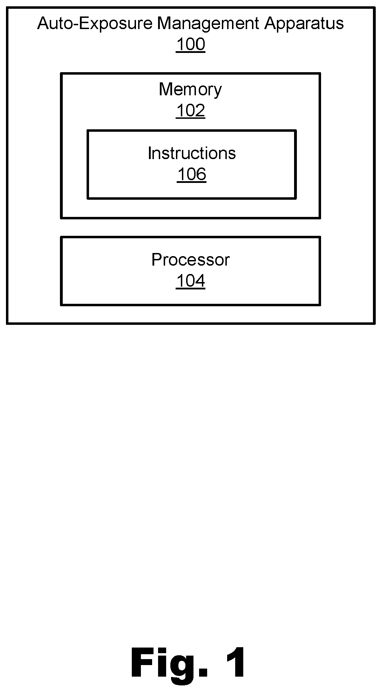

FIG. 1 shows an illustrative auto-exposure management apparatus 100 (apparatus 100) for managing auto-exposure of image frames according to principles described herein. Apparatus 100 may be implemented by computer resources (e.g., servers, processors, memory devices, storage devices, etc.) included within an image capture system (e.g., an endoscopic image capture system, etc.), by computer resources of a computing system associated with an image capture system (e.g., communicatively coupled to the image capture system), and/or by any other suitable computing resources as may serve a particular implementation.

As shown, apparatus 100 may include, without limitation, a memory 102 and a processor 104 selectively and communicatively coupled to one another. Memory 102 and processor 104 may each include or be implemented by computer hardware that is configured to store and/or process computer software. Various other components of computer hardware and/or software not explicitly shown in FIG. 1 may also be included within apparatus 100. In some examples, memory 102 and processor 104 may be distributed between multiple devices and/or multiple locations as may serve a particular implementation.

Memory 102 may store and/or otherwise maintain executable data used by processor 104 to perform any of the functionality described herein. For example, memory 102 may store instructions 106 that may be executed by processor 104. Memory 102 may be implemented by one or more memory or storage devices, including any memory or storage devices described herein, that are configured to store data in a transitory or non-transitory manner. Instructions 106 may be executed by processor 104 to cause apparatus 100 to perform any of the functionality described herein. Instructions 106 may be implemented by any suitable application, software, code, and/or other executable data instance. Additionally, memory 102 may also maintain any other data accessed, managed, used, and/or transmitted by processor 104 in a particular implementation.

Processor 104 may be implemented by one or more computer processing devices, including general purpose processors (e.g., central processing units (CPUs), graphics processing units (GPUs), microprocessors, etc.), special purpose processors (e.g., application specific integrated circuits (ASICs), field-programmable gate arrays (FPGAs), etc.), image signal processors, or the like. Using processor 104 (e.g., when processor 104 is directed to perform operations represented by instructions 106 stored in memory 102), apparatus 100 may perform various functions associated with managing auto-exposure of image frames depicting objects (e.g., extraneous objects) whose influence on the auto-exposure management of the image frame are to be discounted (e.g., objects such as dark instrument shafts present at a scene internal to a body during an endoscopic medical procedure).

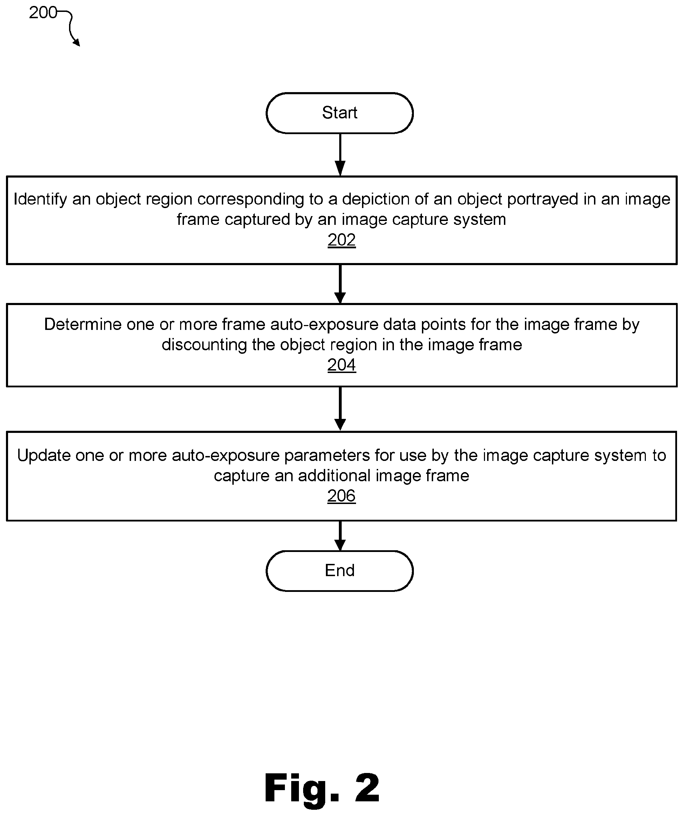

FIG. 2 shows an illustrative auto-exposure management method 200 (method 200) that apparatus 100 may perform to manage auto-exposure of image frames in accordance with principles described herein. While FIG. 2 shows illustrative operations according to one embodiment, other embodiments may omit, add to, reorder, and/or modify any of the operations shown in FIG. 2. In some examples, multiple operations shown in FIG. 2 or described in relation to FIG. 2 may be performed concurrently (e.g., in parallel) with one another, rather than being performed sequentially as illustrated and/or described. One or more of the operations shown in FIG. 2 may be performed by an auto-exposure management apparatus (e.g., apparatus 100), an auto-exposure management system (e.g., an implementation of an auto-exposure management system described below), and/or any implementation thereof.

At operation 202, apparatus 100 may identify an object region in an image frame captured by an image capture system. The object region may correspond to a depiction of an object portrayed in the image frame. For example, the object to which the object region corresponds may be an extraneous object such as any of those described above (e.g., low luminance instrument shafts or ultrasound probes, high luminance gauze or mesh material, etc.) or another object that fits some or all of the criteria described above for an extraneous object (e.g., being a relatively large object, being unlikely to be of interest to the viewer, having significantly different auto-exposure properties than other depicted content, etc.). As will be described in more detail below, apparatus 100 may identify the object region based on local characteristics associated with pixel units (e.g., individual pixels or groups of pixels) included in the image frame, based on global characteristics associated with the image frame, and/or based on any other factors as may serve a particular implementation.

At operation 204, apparatus 100 may determine one or more frame auto-exposure data points for the image frame by discounting the object region in the image frame. For example, by discounting (e.g., completely ignoring or otherwise downplaying the influence of) pixel units identified to be included within the object region, apparatus 100 may determine an auto-exposure value for the image frame (a frame auto-exposure value), an auto-exposure target for the image frame (a frame auto-exposure target), and/or any other frame auto-exposure data point as may serve a particular implementation.

An auto-exposure value will be understood to represent certain auto-exposure-related characteristics (e.g., luminance, signal intensity, chrominance, etc.) of a particular image frame or portion thereof (e.g., region, pixel unit, etc.). For example, apparatus 100 may detect such characteristics by analyzing the image frame captured by the image capture system. A unit auto-exposure value may refer to a luminance determined for a pixel unit. For example, a unit auto-exposure value may be determined as a luminance of an individual pixel or as an average luminance of a group of pixels in an implementation in which pixels are grouped together into pixel cells in a grid, or the like. As another example, a frame auto-exposure value may refer to an average luminance of some or all of the pixel units included within an image frame such that the frame auto-exposure value corresponds to the image frame in an analogous way as a unit auto-exposure value corresponds to a particular pixel unit.

In these examples, it will be understood that the average luminance (and/or one or more other average exposure-related characteristics in certain examples) referred to by an auto-exposure value may be determined as any type of average as may serve a particular implementation. For instance, an average auto-exposure value for an image frame may refer to a mean luminance of pixel units in the image frame, determined by summing respective luminance values for each pixel unit of the image frame and then dividing the sum by the total number of values. As another example, an average auto-exposure value for an image frame may refer to a median luminance of pixel units in the image frame, determined as the central luminance value when the respective luminance values for each pixel unit are ordered by value. As yet another example, an average auto-exposure value for an image frame may refer to a mode luminance of pixel units in the image frame, determined as whichever luminance value, of the respective luminance values for each pixel unit, is most prevalent or repeated most often. In other examples, other types of averages (besides mean, median, or mode) and/or other types of exposure-related characteristics (besides luminance) may also be used to determine an auto-exposure value in any manner as may serve a particular implementation.

An auto-exposure target will be understood to refer to a target (e.g., a goal, a desirable value, an ideal, an optimal value, etc.) for the auto-exposure value of a particular image frame or portion thereof (e.g., region, pixel unit, etc.). Apparatus 100 may determine auto-exposure targets based on the particular circumstances and any suitable criteria, and the auto-exposure targets may relate to the same auto-exposure-related characteristics (e.g., luminance, signal intensity, chrominance, etc.) as are represented by the auto-exposure values. For example, auto-exposure targets may be determined at desirable levels of luminance (or other exposure-related characteristics) such as a luminance level associated with middle gray or the like. As such, a unit auto-exposure target may refer to a desired target luminance determined for a pixel unit (e.g., a desired target luminance for an individual pixel or an average desired target luminance determined for a group of pixels in an implementation in which pixels are grouped together into pixel cells in a grid). As another example, a frame auto-exposure target may refer to an average desired target luminance for some or all of the pixel units included within the image frame, and, as such, may represent an auto-exposure target that corresponds to the image frame in an analogous way as a unit auto-exposure target corresponds to a particular pixel unit. Similarly as described above in relation to how frame auto-exposure values may be determined, frame auto-exposure targets in such examples may be determined by averaging individual unit auto-exposure targets using a mean, median, mode, or other suitable type of averaging technique.

The determining of frame auto-exposure data points such as frame auto-exposure values and/or frame auto-exposure targets at operation 204 may discount the object region identified at operation 202 in any manner as may serve a particular implementation. For example, as will be described in more detail below, in implementations employing weighted averaging of unit auto-exposure data points to determine frame auto-exposure data points, the weight values assigned to pixel units corresponding to the object region may be set to be lower than weight values of pixel units that do not correspond to the object region, or may be completely zeroed out. In these ways, different implementations may be configured to discount the object region by completely ignoring the object region for purposes of auto-exposure management (e.g., entirely excluding the influence of the object region on the auto-exposure management), by reducing the influence of the object region to a more limited extent (e.g., downplaying but not entirely excluding the influence of the object region on the auto-exposure management), or by doing both of these in accordance with confidence levels associated with each pixel unit.

At operation 206, apparatus 100 may update (e.g., adjust or maintain) one or more auto-exposure parameters for use by the image capture system to capture one or more additional image frames. In some examples, apparatus 100 may update the one or more auto-exposure parameters based on auto-exposure values, auto-exposure targets, and/or other auto-exposure data points of the pixels of the image frame as those data points have been determined (e.g., at operation 204). For instance, assuming that apparatus 100 has determined a frame auto-exposure value and/or a frame auto-exposure target, apparatus 100 may update the one or more auto-exposure parameters at operation 206 based on the frame auto-exposure value and/or frame auto-exposure target. For example, apparatus 100 may determine an auto-exposure gain for the image frame (a frame auto-exposure gain) based on the frame auto-exposure value and frame auto-exposure target, and may perform the updating of the one or more auto-exposure parameters based on the frame auto-exposure gain.

Apparatus 100 may update the auto-exposure parameters by either adjusting the parameters or maintaining the parameters as appropriate based on the auto-exposure gain. In this way, the image capture system may capture one or more additional image frames (e.g., subsequent image frames in an image frame sequence being captured) using auto-exposure parameters (e.g., exposure time parameters, shutter aperture parameters, illumination intensity parameters, image signal analog and/or digital gains, etc.) that may reduce the difference between auto-exposure values detected for those additional image frames and auto-exposure targets desirable for those additional image frames. Accordingly, the additional image frames may be captured with more desirable exposure characteristics than might be captured without such adjustments, and users of apparatus 100 may experience a superior image (e.g., an image that shows details of content other than an extraneous object at a desired brightness level, etc.).

Apparatus 100 may be implemented by one or more computing devices or by computing resources of a general purpose or special purpose computing system such as will be described in more detail below. In certain embodiments, the one or more computing devices or computing resources implementing apparatus 100 may be communicatively coupled with other components such as an image capture system used to capture the image frames that apparatus 100 is configured to process. In other embodiments, apparatus 100 may be included within (e.g., implemented as a part of) an auto-exposure management system. Such an auto-exposure management system may be configured to perform all the same functions described herein to be performed by apparatus 100 (e.g., including the operations of method 200, described above), but may further incorporate additional components such as the image capture system so as to also be able to perform the functionality associated with these additional components.

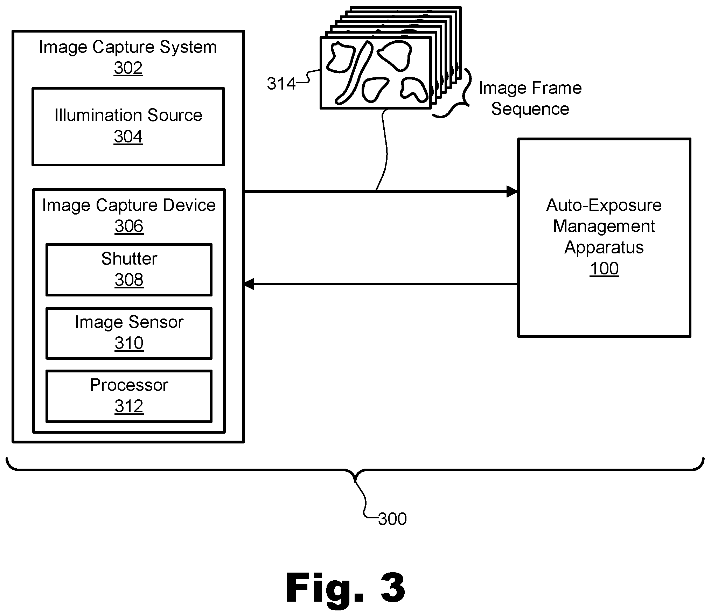

FIG. 3 shows an illustrative auto-exposure management system 300 (system 300) for managing auto-exposure of image frames. As shown, system 300 may include an implementation of apparatus 100 together with an image capture system 302 that includes an illumination source 304 and an image capture device 306 that incorporates a shutter 308, an image sensor 310, and a processor 312 (e.g., one or more image signal processors implementing an image signal processing pipeline). Within system 300, apparatus 100 and image capture system 302 may be communicatively coupled to allow apparatus 100 to direct image capture system 302 in accordance with operations described herein, as well as to allow image capture system 302 to capture and provide to apparatus 100 an image frame sequence 314 and/or other suitable captured image data. Components of image capture system 302 will each be described in more detail below.

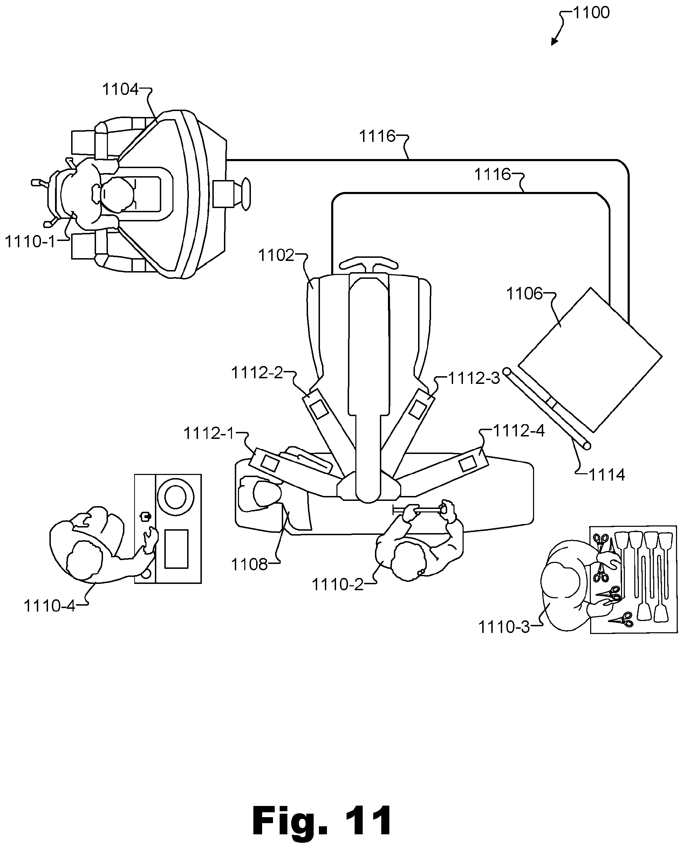

As has been mentioned, while principles described herein may be applied to a wide variety of imaging scenarios, many examples explicitly described herein relate to medical procedures that may be performed using a computer-assisted medical system such as will be described in more detail below in relation to FIG. 11. In such examples, the scene for which images are being captured may include an internal view of a body on which the medical procedure is being performed (e.g., a body of a live animal, a human or animal cadaver, a portion of human or animal anatomy, tissue removed from human or animal anatomies, non-tissue work pieces, training models, etc.). As such, system 300 or certain components thereof (e.g., image capture system 302) may be integrated with (e.g., implemented by imaging and computing resources of) a computer-assisted medical system and the objects that are to be discounted in the auto-exposure management may include objects associated with the computer-assisted medical system or medical procedure (e.g., instruments included within the computer-assisted medical system, gauze or mesh material used for the medical procedure, etc.).

In certain such examples, apparatus 100 may be configured to identify the object region based on the difference in color of the extraneous objects and the tissue featured in the internal view of the body. For instance, apparatus 100 may determine, for environmental imagery of the scene depicted in the image frame (e.g., for imagery of elements of the scene other than extraneous objects such as instruments, gauze, or other objects foreign to the internal body), a color gamut encompassing a range of red colors corresponding to blood and tissue visible in the internal view of the body. Then, based on one or more chrominance characteristics of pixel units included in the image frame, apparatus 100 may identify, within the image frame, an object region corresponding to a depiction of an object portrayed in the image frame by performing operations including, for example, determining whether the chrominance characteristics of the pixel units are included within the color gamut for the environmental imagery of the scene.

illumination source 304 may be implemented to source any type of illumination (e.g., visible light, infrared or near-infrared light, fluorescence excitation light, etc.) and may be configured to interoperate with image capture device 306 within image capture system 302. For example, illumination source 304 may provide a certain amount of illumination to a scene to facilitate image capture device 306 in capturing optimally illuminated images of the scene.

image capture device 306 may be implemented by any suitable camera or other device configured to capture images of a scene. For instance, in a medical procedure example, image capture device 306 may be implemented by an endoscopic image capture device configured to capture image frame sequence 314, which may include an image frame depicting a view (e.g., an internal view) of the body undergoing the medical procedure. As shown, image capture device 306 may include components such as shutter 308, image sensor 310, and processor 312.

Image sensor 310 may be implemented by any suitable image sensor, such as a charge coupled device (CCD) image sensor, a complementary metal-oxide semiconductor (CMOS) image sensor, or the like.

Shutter 308 may interoperate with image sensor 310 to assist with the capture and detection of light from the scene. For example, shutter 308 may be configured to expose image sensor 310 to a certain amount of light for each image frame captured. Shutter 308 may comprise an electronic shutter and/or a mechanical shutter. Shutter 308 may control how much light image sensor 310 is exposed to by opening to a certain aperture size defined by a shutter aperture parameter and/or for a specified amount of time defined by an exposure time parameter. As will be described in more detail below, these shutter-related parameters may be included among the auto-exposure parameters that apparatus 100 is configured to update.

Processor 312 may be implemented by one or more image signal processors configured to implement at least part of an image signal processing pipeline. Processor 312 may process auto-exposure statistics input (e.g., by tapping the signal in the middle of the pipeline to detect and process various auto-exposure data points and/or other statistics), perform optics artifact correction for data captured by image sensor 310 (e.g., by reducing fixed pattern noise, correcting defective pixels, correcting lens shading issues, etc.), perform signal reconstruction operations (e.g., white balance operations, demosaic and color correction operations, etc.), apply image signal analog and/or digital gains, and/or perform any other functions as may serve a particular implementation. Various auto-exposure parameters may dictate how the functionality of processor 312 is to be performed. For example, auto-exposure parameters may be set to define the analog and/or digital gains processor 312 applies, as will be described in more detail below.

In some examples, an endoscopic implementation of image capture device 306 may include a stereoscopic endoscope that includes two full sets of image capture components (e.g., two shutters 308, two image sensors 310, etc.) to accommodate stereoscopic differences presented to the two eyes (e.g., left eye and right eye) of a viewer of the captured image frames. Conversely, in other examples, an endoscopic implementation of image capture device 306 may include a monoscopic endoscope with a single shutter 308, a single image sensor 310, and so forth.

Apparatus 100 may be configured to control various auto-exposure parameters of image capture system 302 and may adjust such auto-exposure parameters in real time based on incoming image data captured by image capture system 302. As mentioned above, certain auto-exposure parameters of image capture system 302 may be associated with shutter 308 and/or image sensor 310. For example, apparatus 100 may direct shutter 308 in accordance with an exposure time parameter corresponding to how long the shutter is to allow image sensor 310 to be exposed to the scene, a shutter aperture parameter corresponding to an aperture size of shutter 308, or any other suitable auto-exposure parameters associated with shutter 308. Other auto-exposure parameters may be associated with aspects of image capture system 302 or the image capture process unrelated to shutter 308 and/or sensor 310. For example, apparatus 100 may adjust an illumination intensity parameter of illumination source 304 that corresponds to an intensity of illumination provided by illumination source 304, an illumination duration parameter corresponding to a time period during which illumination is provided by illumination source 304, or the like. As yet another example, apparatus 100 may adjust gain parameters corresponding to one or more analog and/or digital gains (e.g., analog gains, bayer gains, RGB gains, etc.) applied by processor 312 to image data (e.g., luminance data) generated by image sensor 310.

Any of these or other suitable parameters, or any combination thereof, may be updated and/or otherwise adjusted by apparatus 100 for subsequent image frames based on an analysis of the current image frame. For instance, in one example where the frame auto-exposure gain (e.g., the frame auto-exposure target divided by the frame auto-exposure value) is determined to be 6.0, various auto-exposure parameters could be set as follows: 1) a current illumination intensity parameter may be set to 100% (e.g., maximum output); 2) an exposure time parameter may be set to 1/60^{th} of a second (e.g., 60 fps); 3) an analog gain may be set to 5.0 (with a cap of 10.0); 4) a bayer gain may be set to 1.0 (with a cap of 3.0); and 5) an RGB gain may be set to 2.0 (with a cap of 2.0). With these settings, the gain is distributed across the analog gain (10.0/5.0 = 2.0), bayer gain (3.0/1.0 = 3.0), and RGB gain (2.0/2.0 = 1.0) to establish the desired 6.0 total auto-exposure gain (3.0 * 2.0 * 1.0 = 6.0) for the frame.

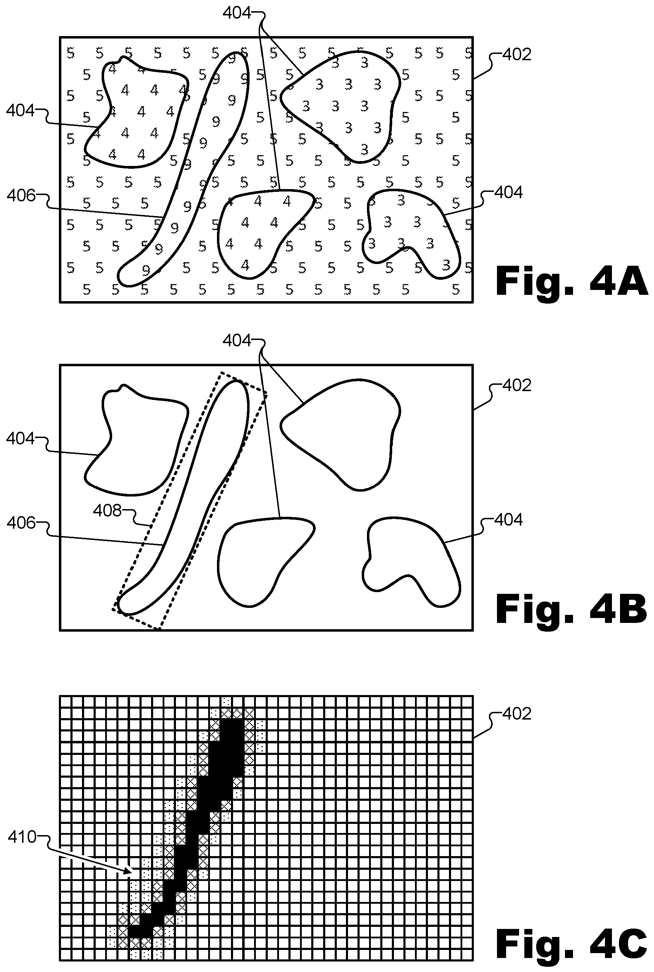

FIGS. 4A-4C show various aspects of an illustrative image frame 402 that depicts an illustrative object whose influence on the auto-exposure management of an image frame sequence may be discounted by apparatus 100. For example, image frame 402 may be an image frame that is captured by system 300 and included as one of the image frames of image frame sequence 314. Image frame 402 is shown to depict, in front of a background, various objects 404 that may be of interest to a viewer of image frame 402 and a particular object 406 that may be an extraneous object that is to be discounted (e.g., because it is unlikely to be of interest to the viewer, because it has significantly different luminance than objects 404 and the background, etc.). For instance, in a medical procedure example, each of objects 404 may represent anatomical objects featured on an anatomical background (e.g., a background featuring blood, tissue, etc., that is similar in luminance to the anatomical objects), while object 406 may represent an instrument shaft, piece of gauze, or another such extraneous object that it may be desirable for the auto-exposure management to discount. While only a single extraneous object 406 is illustrated in FIGS. 4A and 4B and described in many examples herein, it will be understood that a plurality of extraneous objects may be discounted from an image frame by discounting each object in the same manner described herein for object 406 and/or other individual extraneous objects described herein. For example, the image frame 402 may depict a plurality of instrument shafts (e.g., from two different instruments, three different instruments, etc.), may depict an instrument shaft and a piece of gauze, etc.

FIG. 4A illustrates how local characteristics may be employed to identify an object region corresponding to a depiction of object 406 portrayed in image frame 402. For example, the local characteristics may include chrominance characteristics (e.g., color properties) or luminance characteristics (e.g., brightness properties) associated with individual pixel units included within image frame 402, as well as to corresponding properties expected for other content of image frame 402 and/or for object 406.

While FIG. 4A is depicted as a black and white drawing, various digits 1-9 used to fill the various objects and background areas depicted in image frame 402 will be understood to represent different colors or, alternatively, different brightness values. In this digit-based chrominance/luminance notation, digits closer to one another (e.g., 1 and 2, 8 and 9, etc.) will be understood to represent similar colors (e.g., red and red-orange, green and green-yellow, etc.) or similar brightness levels, while digits farther away from one another (e.g., 1 and 8, 2 and 9, etc.) will be understood to represent more distinct colors (e.g., red and green, blue and orange, etc.) or different brightness levels. In some examples, this digit-based notation may be interpreted to wrap around such that digit 1 is considered to be adjacent to digit 9 and color or brightness represented by digit 1 is similar to color or brightness represented by digit 9.

Referring to FIG. 4A in terms of chrominance characteristics, the color of object 406 (which is shown to be a color denoted by digit 9) may be notably different from the color of objects 404 and the background (which are shown to be similar colors in the range of digits 3-5). One local characteristic used to identify an object region corresponding to the depiction of object 406 may be whether the color of object 406 is proximate to a predetermined color that may be expected for known extraneous objects (e.g., a neutral color such as a metallic gray or black for an instrument shaft or sheath expected to commonly be present at the scene of a medical procedure, etc.). If digit 9 represents neutral colors (e.g., blacks, grays, etc.) while digits 3-5 represent different shades of red (e.g., shades commonly associated with tissue, blood, and/or bloody tissue present at an internal scene within a body), apparatus 100 may identify the object region at least partially based on how similar the color of object 406 is to the expected neutral color of an instrument.

As another example of a local characteristic used to identify the object region, apparatus 100 may determine whether the color of object 406 is significantly different from an average color of the scene (e.g., an average color of the entire scene including the object, an average color of the environmental imagery of the scene, etc.) or from an expected color for the environmental imagery of the scene that is to be accounted for in the auto-exposure management (e.g., an expected color for tissue and anatomical objects such as a red color associated with tissue or blood). For example, if it is again assumed that digit 9 represents a neutral color while digits 3-5 represent reddish shades, apparatus 100 may identify the object region at least partially based how different the color of object 406 is from the expected red color of objects 404 and the background. If the digit-based notation is interpreted to represent luminance characteristics rather than chrominance characteristics, similar deductions may be made in terms of luminance to further facilitate identification of the object region.

FIG. 4B illustrates how global characteristics may be employed to identify the object region corresponding to the depiction of object 406 portrayed in image frame 402. For example, the global characteristics may relate to object tracking that is performed with respect to object 406. Computer vision techniques, kinematic tracking techniques (e.g., in an example where object 406 is a robotically-controlled instrument, etc.), and/or other ways of tracking an object within an image frame sequence may be employed by apparatus 100 or by a system independent from apparatus 100. In either case, apparatus 100 may obtain data that is generated based on the object tracking to identify the position of object 406. For example, the object tracking data may represent a bounding box 408 or the like that indicates where the object tracking has determined that object 406 is currently located.

By analyzing local and/or global characteristic such as those illustrated in FIG. 4A and 4B, apparatus 100 may successfully and efficiently identify an object region corresponding to the depiction of object 406 in image frame 402. Analyses involving such local and/or global characteristics will be described in more detail below. Once the object region is identified, apparatus 100 may be configured to discount the identified object region in the determination of frame auto-exposure data points (e.g., a frame auto-exposure value and/or a frame auto-exposure target to be used as a basis for auto-exposure management of subsequent image frames).

To illustrate, FIG. 4C shows how pixel units associated with an identified object region 410 may be discounted as part of the determination of frame auto-exposure data points for image frame 402. As mentioned above, a pixel unit may refer to either an individual pixel or a group of pixels as may serve a particular implementations or image frame. For example, an image frame such as image frame 402 may be divided into cells of a grid and each cell may include one or more individual pixels such that each pixel unit refers to a cell of the grid with its respective pixel(s). In one case, each cell of a grid into which the image frame is divided may include only one individual pixel, resulting in the pixel units mentioned above that have only an individual pixel. In other cases, each cell of the grid may include a group of several pixels (e.g., 4 pixels, 16 pixels, 512 pixels, etc.), resulting in the pixel units mentioned above that include a group of pixels.

As shown in FIG. 4C, image frame 402 includes a grid of many small squares each representing a pixel unit of image frame 402 (e.g., an individual pixel or group of pixels). Each pixel unit in FIG. 4C is shaded according to a weight value assigned to the pixel unit, as will be described in more detail below. For example, pixel units without any shading (e.g., the majority of the pixel units which are still white) may be understood to be assigned weight values that allow the pixel units to be fully accounted for by the auto-exposure management, while pixel units that include at least some degree of shading (e.g., the pixel units in object region 410) may be understood to be assigned weight values that cause the pixel units to be discounted by the auto-exposure management.

Different shadings shown in FIG. 4C may represent different weight values and, as described in more detail below, may correspond to a confidence level of apparatus 100 that the pixels are to be identified within object region 410. For example, as shown, lighter shadings may be assigned to peripheral pixel units around object 406 because these pixel units may partially represent object 406 and partially represent other imagery (e.g., the background), and/or because it may be difficult for apparatus 100 to determine with 100% confidence whether or not a given pixel unit is associated with object 406 based on the local and global characteristics and/or other factors that may be used. As a result of the different weight values, apparatus 100 may discount different parts of object region 410 to different extents when performing the auto-exposure management (e.g., when determining the frame auto-exposure data points and updating the auto-exposure parameters based on the frame auto-exposure data points). For example, for the internal portions of object region 410 shaded in black, apparatus 100 may completely ignore these pixel units in the auto-exposure management, whereas, for the peripheral portions of object region 410 shaded with cross-hatching or dots, apparatus 100 may discount the influence of these pixel units to different extents (e.g., to a greater extent for the pixel units shaded with cross-hatching and to a lesser extent for the pixel units shaded with dots, etc.).

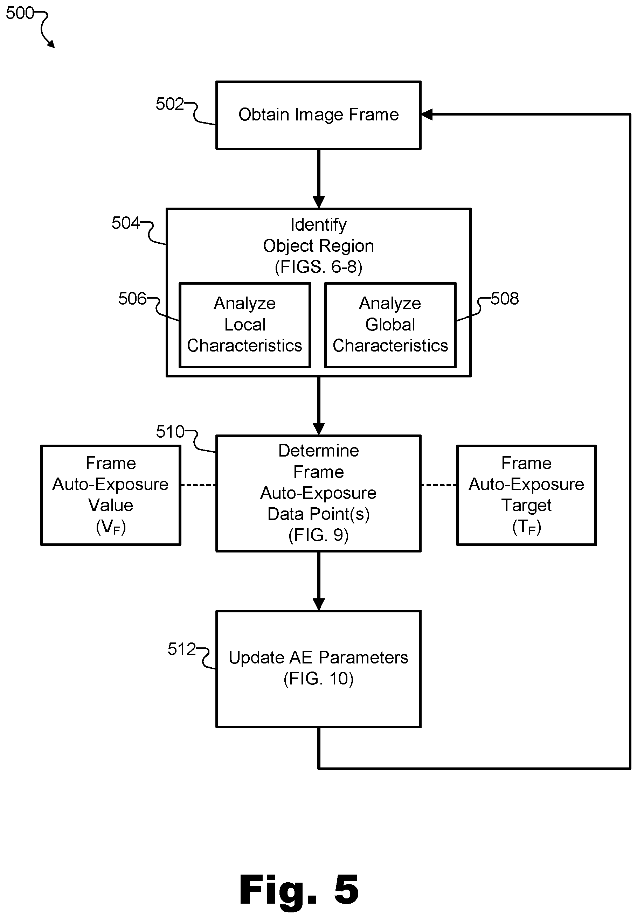

FIG. 5 shows an illustrative flow diagram 500 for managing auto-exposure of image frames using, for example, an implementation of apparatus 100, method 200, and/or system 300. As shown, flow diagram 500 illustrates various operations 502-512, which will each be described in more detail below. It will be understood that operations 502-512 represent one embodiment, and that other embodiments may omit, add to, reorder, and/or modify any of these operations. As will be described, various operations 502-512 of flow diagram 500 may be performed for one image frame or multiple image frames (e.g., each image frame) in an image frame sequence. It will be understood that, depending on various conditions, not every operation might be performed for every frame, and the combination and/or order of operations performed from frame to frame in the image frame sequence may vary.

At operation 502, an image frame captured by an image capture system may be obtained (e.g., accessed, loaded, captured, generated, etc.). As previously explained, in certain examples, the image frame may be an image frame depicting one or more objects, including an extraneous object whose influence on auto-exposure management of the image frame sequence is to be discounted. For example, the obtained image frame may be similar to image frame 402 described above and the extraneous object may be object 406. Operation 502 may be performed in any suitable way, such as by accessing the image frame from an image capture system (e.g., in the case that operation 502 is being performed by an implementation of apparatus 100 that is communicatively coupled to an image capture system) or by using an integrated image capture system to capture the image frame (e.g., in the case that operation 502 is being performed by an implementation of system 300 that includes integrated image capture system 302).

At operation 504, apparatus 100 may identify an object region within the image frame obtained at operation 502 based on any suitable factors as may serve a particular implementation. For example, the identifying of the object region at operation 504 may be performed based on one or more local characteristics associated with pixel units included in the image frame, based on one or more global characteristics associated with the image frame, or based on a combination of both (e.g., a combination of a local characteristic associated with a pixel unit included in the image frame and a global characteristic associated with the image frame). To this end, as shown, operation 504 may include either or both of operation 506, in which apparatus 100 analyzes local characteristics of pixel units of the image frame, and operation 508, in which apparatus 100 analyzes global characteristics of the image frame. As indicated in FIG. 5, FIGS. 6-8 further illustrate various aspects of how an object region may be identified at operation 504.

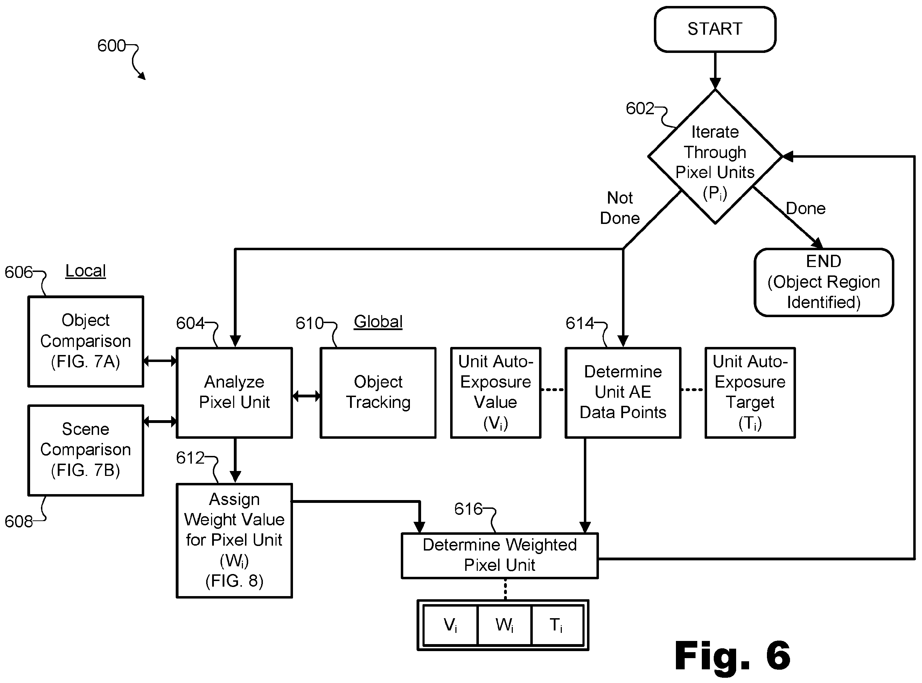

FIG. 6 shows an illustrative flow diagram 600 for identifying an object region within an illustrative image frame such as an image frame obtained at operation 502. As shown, flow diagram 600 includes a plurality of operations 602-616 that may be performed between when apparatus 100 begins performing operation 504 (labeled START) and when flow diagram 600 is complete and the object region has been identified (labeled END).

At operation 602, apparatus 100 may iterate through each pixel unit of an image frame or portion of an image frame. For each pixel unit Pᵢ, the pixel unit may be analyzed at operation 604 (e.g., which may include performing one or more of operations 606-610) and a weight value (Wᵢ) may be assigned at operation 612. Additionally (e.g., before, after, or concurrently with the performance of operations 604 and 612), unit auto-exposure data points such as unit auto-exposure values (Vᵢ) and/or unit auto-exposure targets (Tᵢ) for each pixel unit Pᵢ may be determined at operation 614. Then, at operation 616, a weighted pixel unit may be determined for each pixel unit Pᵢ based on the results of operations 604-614 that have been performed. As shown, apparatus 100 may continue processing each pixel unit in this manner as long as there are still pixel units of the image frame that have not yet been processed (Not Done), and may end when all of the pixel units of the image frame have been iterated through at operation 602 (Done). In certain examples, rather than iterating through all of the pixel units of the image frame, a certain region of the image frame (e.g., a central region of the image frame such as a central 50% of the image frame, a central 80% of the image frame, etc.) may be accounted for while another region of the image frame (e.g., a peripheral region of the image frame such as an outer 50% of the image frame, an outer 20% of the image frame, etc.) may be ignored for purposes of auto-exposure management. In such examples, operation 602 may finish iterating (Done) when all the pixels of the region that is to be accounted for (e.g., the central region) have been iterated through.

At operation 604, apparatus 100 may analyze the current pixel unit Pᵢ to determine whether the pixel unit corresponds to a depiction of a particular object (e.g., an extraneous object) within the image frame, or, in certain implementations, a confidence level (e.g., on a scale of 0% confidence to 100% confidence or on another suitable scale such as a high-medium-low confidence scale, etc.) that the pixel unit corresponds to the depiction of the particular object. As shown, to accomplish this analysis at operation 604, apparatus 100 may perform any or all of operations 606 through 610 or other suitable operations not explicitly shown to help achieve the same end.

Operations 606 and 608 are each shown in FIG. 6 to fall under a Local category because these operations determine the confidence for the pixel unit largely or entirely based on one or more local characteristics associated with the pixel unit being analyzed. Local characteristics may include various types of pixel unit characteristics as described above in relation to FIG. 4A. For instance, the one or more local characteristics associated with the pixel unit may include a luminance characteristic of the pixel unit (e.g., an average brightness of the pixel unit, etc.). As another example, the one or more local characteristics associated with the pixel unit may include a chrominance characteristic of the pixel unit (e.g., an average color of the pixel unit, etc.). In some implementations, the one or more local characteristics associated with the pixel unit may include both chrominance and luminance characteristics of the pixel units. For instance, a confidence that a pixel unit depicts an extraneous object may be determined based on comparisons of both chrominance characteristics and luminance characteristics in the ways described below.

At operation 606, the one or more local characteristics of the pixel unit may be compared to corresponding characteristics associated with the object for which the object region is being identified (e.g., an instrument shaft that has a low luminance and a neutral, metallic color in one particular example). In such examples, the identifying of the object region may be based on a comparison of a luminance characteristic of the pixel unit to a luminance characteristic associated with the object (e.g., to determine if the pixel unit is similarly dark as expected for the instrument shaft object) and/or based on a comparison of a chrominance characteristic of the pixel unit to a chrominance characteristic associated with the object (e.g., to determine if the pixel unit is similarly neutral in color as expected for the instrument shaft object).

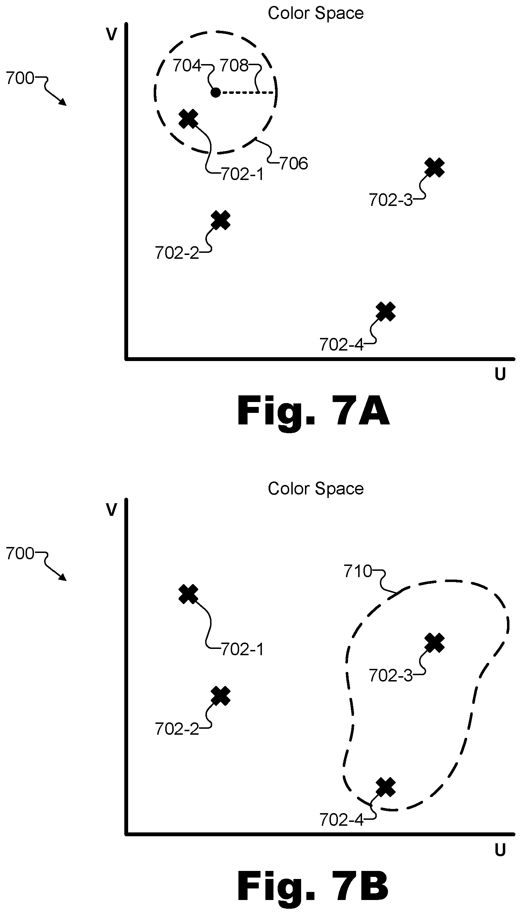

To further illustrate operation 606, FIG. 7A shows illustrative geometric characteristics that may be analyzed within an illustrative color space 700 to help identify the object region within the image frame. As part of operation 606 (or in a separate operation not explicitly shown), apparatus 100 may normalize and decompose color data associated with a current pixel unit Pᵢ to distinguish chrominance characteristics of the color data from luminance characteristics of the color data. For example, the normalized color data for each pixel unit may be decomposed from a Red-Green-Blue (RGB) color space (in which chrominance and luminance characteristics for each pixel are jointly represented by a Red value, a Green value, and a Blue value) into a different color space that accounts for primary, secondary, and/or tertiary colors and that separately accounts for luminance characteristics. As one example shown in FIG. 7A, the decomposing of the color data may include converting the color data from an RGB color space to a YUV color space. It will be understood that, in other implementations, the decomposing of the color data could include converting the color data to a Cyan-Magenta-Yellow-blacK-Red-Green-Blue (CMYKRGB) color space, a CIELAB color space, or another suitable color space that allows for the chrominance characteristics to be conveniently analyzed independently from luminance characteristics.

In FIG. 7A, several different points 702 (e.g., points 702-1 through 702-4) are plotted within a UV coordinate space associated with YUV color space 700 to represent respective colors of several pixel unit examples (or, more generally, to represent respective chrominance characteristics of the pixel unit examples). For each example pixel unit represented by points 702 in UV space, it will be understood that a separate luminance value Y may also be associated with the pixel unit (e.g., to represent the pixel unit's luminance characteristic), although this is not shown in FIG. 7A.

To compare the chrominance characteristic of a pixel unit to a chrominance characteristic of a particular object (e.g., an extraneous object that is to be discounted in the auto-exposure management), apparatus 100 may apply geometric principles within the UV coordinate plane. For example, if the chrominance characteristic of the particular object is represented by a point 704 in the UV coordinate space, apparatus 100 may calculate a distance (e.g., a Euclidean distance) between a particular point 702 and point 704 to determine an objective and quantitative measure of how similar or dissimilar the color of the pixel unit is to the color of the particular object. For example, because point 702-1 is relatively close to point 704, a comparison of points 702-1 and 704 may indicate that the chrominance characteristics represented by these points are quite similar (as shown by the relatively close proximity of the points), while a comparison of points 702-4 and 704 may indicate that the chrominance characteristics represented by these points are quite different (as shown by the relatively far distance between the points).

In some examples, the object comparison of operation 606 may include determining whether the distance between points exceeds or does not exceed a particular threshold. Such determinations may be used in assigning weight values for a given pixel unit, as will be described in more detail below. To illustrate, a threshold 706 is drawn around point 704 with a radius 708. Any point 702 that is close enough to point 704 to be within the circle of threshold 706 may be considered to exceed or meet threshold 706. For example, the chrominance characteristic represented by point 702-1 is similar enough to the chrominance characteristic represented by point 704 to meet threshold 706. Conversely, any point 702 that is far enough from point 704 to be outside of the circle of threshold 706 may be considered not to exceed, or to fail to meet, threshold 706. For example, the chrominance characteristic represented by points 702-2 through 702-4 are each dissimilar enough from the chrominance characteristic represented by point 704 to fail to meet threshold 706.

At operation 608 of FIG. 6, the one or more local characteristics of the pixel unit may be compared to corresponding characteristics associated with scene content other than the object (e.g., environmental imagery such as bright red blood, tissue, and/or other scene content present in the internal view of the body together with the dark, neutral colored instrument shaft). As such, in these examples, the identifying of the object region may be based on a comparison of a luminance characteristic of the pixel unit to a luminance characteristic associated with the environmental imagery of the scene (e.g., to determine if the pixel unit is similarly bright as expected for the blood and tissue depicted at the scene) and/or based on a comparison of a chrominance characteristic of the pixel unit to a chrominance characteristic associated with the environmental imagery of the scene (e.g., to determine if the pixel unit is similarly red in color as expected for the blood and tissue depicted at the scene).

To further illustrate operation 608, FIG. 7B shows additional illustrative geometric characteristics that may be analyzed within illustrative color space 700 that was described above in relation to FIG. 7A. FIG. 7B shows the same points 702 within the UV coordinate space of YUV color space 700. Additionally, FIG. 7B shows a color gamut 710 that apparatus 100 may determine to be associated with environmental imagery of the scene (e.g., a color gamut encompassing a range of red colors corresponding to blood and tissue visible in the internal view of the body for a medical procedure example). For instance, color gamut 710 may be predefined and accessed by apparatus 100 (e.g., loaded from memory as a profile for a particular scenario such as a surgical procedure, etc.) or may be determined based on an average color gamut of the scene or the environmental imagery thereof as depicted by the image frame or previous image frames that have been analyzed. While color gamut 710 is shown as an irregular shape, it will be understood that color gamut 710 may be implemented in other examples as a circle, a polygon, or any other suitable shape as may serve a particular implementation.

To compare the chrominance characteristic of a pixel unit to chrominance characteristics of other scene content (e.g., chrominance characteristics of environmental imagery at the scene including objects and content other than the extraneous object that is to be discounted), apparatus 100 may again apply geometric principles within the UV coordinate plane. For example, if the chrominance characteristics of the environmental imagery of the scene are represented by color gamut 710 in the UV coordinate space, apparatus 100 may determine whether a particular point 702 is included within color gamut 710. For example, because points 702-3 and 702-4 are positioned within the boundary of color gamut 710, these points may be determined to be likely to represent environmental imagery of the scene (e.g., to not depict the extraneous object). Conversely, because points 702-1 and 702-2 are positioned well outside the boundary of color gamut 710, these points may be determined to be likely not to represent environmental imagery of the scene (e.g., to be more likely to depict the extraneous object).

Luminance characteristics for different pixel units may be analyzed and compared to known luminance characteristics for extraneous objects (e.g., instrument shafts) or environmental imagery (e.g., blood and tissue) present at a scene in a similar way as has been described for chrominance characteristics in relation to FIGS. 7A and 7B. However, rather than being represented on a two-dimensional coordinate plane as is the case for chrominance characteristics, luminance characteristics, distances, thresholds, ranges (analogous to color gamuts), and so forth, may all be determined and represented on a one-dimensional number line.

Returning to FIG. 6, operation 610 may be performed in addition or as an alternative to operations 606 and/or 608. At operation 610, apparatus 100 may perform object tracking to determine a position of an object (e.g., an extraneous object that is to be discounted) within a scene depicted in the image frame. Operation 610 is shown to fall under a Global category because operation 610 is configured to help determine the confidence for each pixel unit largely or entirely based on one or more global characteristics associated with the image frame being analyzed. As described above in relation to FIG. 4B, one or more global characteristics may include an object position characteristic determined based on object tracking data received from an object tracking system that tracks a position of the particular object within the scene depicted in the image frame.

The object tracking system may be implemented by any suitable system and may operate in any manner as may serve a particular implementation. For instance, in an example where the object to be tracked is an instrument controlled by a robotic arm within a computer-assisted medical system, an object tracking system may be integrated within the computer-assisted medical system (e.g., along with an implementation of apparatus 100 and/or system 300) and may track the position of the object based on kinematic data associated with movements of the robotic arm. Kinematic data may be continuously generated and tracked by a computer-assisted medical system based on movements that each robotic arm is directed to make and sensors indicating how the robotic arm is positioned. Such data may therefore be translated to indicate, for example, where an instrument controlled by one robotic arm is positioned in space relative to an imaging device (e.g., an endoscope) controlled by another robotic arm (or by the same robotic arm in certain implementations).

In the same or other examples, the object tracking system may track the position of the object based on computer vision techniques applied to image frames of an image frame sequence that includes the image frame. For example, object recognition techniques (e.g., including techniques that leverage machine learning or other types of artificial intelligence) performed to recognize an instrument in a medical procedure, or to recognize another type of object in another context, may also be used to help keep track of where the object is positioned in the scene.

Object tracking data determined using kinematic, computer vision, or other suitable techniques may be used, instead of or in addition to data derived from local-based techniques described above, as a basis for apparatus 100 to determine a confidence level for whether each pixel unit corresponds to the depiction of the object in the image frame. As such, object tracking data may be determined and represented in any suitable manner. As one example, an object tracking system may output coordinates of a bounding box that surrounds the depiction of the object in the image frame (e.g., such as bounding box 408 in FIG. 4B). As another example, the object tracking system may output a semantic segmentation map of various objects (e.g., instruments, anatomical objects, etc.) depicted at the scene and that includes semantic segmentation data for the object that is to be discounted in the auto-exposure management.

At operation 612, each of the plurality of pixels of the image frame or region thereof (e.g., each pixel unit Pᵢ) may be assigned a respective weight value (Wᵢ) based on the analysis of the pixel unit performed at operation 604 and/or based on other suitable weighting factors (e.g., a spatial position of the pixel unit within the image frame, etc.). Respective weight values assigned at operation 612 may be indicative of respective confidence levels that the pixel units are included in the depiction of an extraneous object (e.g., confidence levels that each particular pixel unit corresponds, or does not correspond, to the depiction of the extraneous object) as indicated by any local-based pixel comparison operation such as operations 606 and 608, by any global-based object tracking operation such as operation 610, or by any other confidence analysis as may be performed as part of operation 604 in a particular implementation. For example, the probability that a pixel unit depicts an extraneous object may be estimated by taking local and global characteristics into account using a Bayer formula or in another suitable way.

In some examples, the weight values may also be determined in a manner that accounts for how likely each pixel unit is to be within an area of focus of a viewer of the image frame, and, thus, how relatively important each pixel unit is considered to be with respect to other pixel units in the image frame. For example, in certain implementations, it may be assumed that the viewer is likely to focus attention near a center of the image frame, so a weight value assigned to each pixel unit may be at least partially based on a proximity of the pixel unit to the center of the image frame (e.g., with higher weight values indicating closer proximity to the center and lower weight values indicating a farther distance from the center). As another example, an implementation could include an eye tracking feature to determine in real time what part of the image frames the viewer is focusing on, and weight values assigned to each pixel unit may be at least partially based on a proximity of the pixel unit to the detected real-time area of focus (e.g., rather than or in addition to the center of the image frame). In still other examples, weight values assigned to pixel units may be influenced by other spatial-position-based criteria (e.g., proximity to another assumed area of focus within the image frame other than the center, etc.) or non-spatial-position-based criteria. Alternatively, each pixel unit may be treated as equally important regardless of its spatial position in certain examples, such that the weight value is entirely based on the confidence analysis and not the spatial position of the pixel unit.

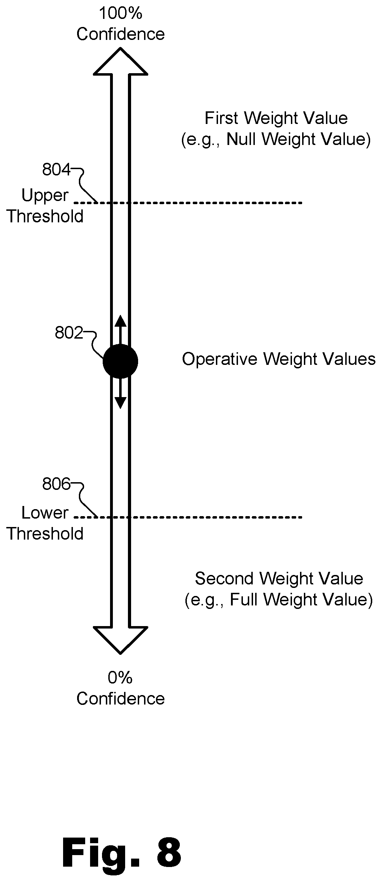

FIG. 8 shows an illustrative range of weight values that may be assigned to a pixel unit at operation 612 to indicate a confidence level that the pixel unit is included in a depiction of an extraneous object within an image frame. Specifically, a weight value 802 is depicted as being able to slide on a confidence scale from 100% confidence to 0% confidence based on the pixel unit analyses performed at operation 604. It will be understood that weight value 802 represents only a confidence-based aspect of an overall weight value that may be assigned to a particular pixel unit, and that one or more other aspects (e.g., a spatial-position-based aspect such as described above) may also be accounted for in assigning an overall weight value to a particular pixel unit.

If the analysis performed at operation 604 (e.g., analyses associated with any of operations 606-610) results in a very high level of confidence, weight value 802 may cross an upper threshold 804 and be assigned a first weight value. For example, the first weight value may be a minimum weight value (e.g., 0%) referred to herein as a null weight value. The first weight value may cause the auto-exposure management to discount (e.g., completely ignore) this pixel unit due to the high level of confidence that the pixel unit depicts the object that is to be discounted. Conversely, if the analysis performed at operation 604 results in a very low level of confidence, weight value 802 may cross a lower threshold 806 and be assigned a second weight value. For example, the second weight value may be a maximum weight value (e.g., 100%) referred to herein as a full weight value. The second weight value may cause the auto-exposure management to give significant or full weight to this pixel unit due to confidence that the pixel unit does not depict the object that is to be discounted. If the confidence level determined by operation 604 is between these thresholds (e.g., is neither very high nor very low), weight value 802 may be assigned an operative weight value that is between the first weight value and the second weight value (e.g., a value greater than 0% and less than 100%). For example, an operative weight value may cause the auto-exposure management to account for this pixel unit to a limited extent due to a likelihood that the pixel unit partially depicts the object that is to be discounted (e.g., depicts part of an edge of the object, etc.) or due to a lack of certainty about whether the pixel unit depicts the object or not.

Each analysis associated with operation 604 may contribute to the overall weight value assigned to a pixel unit. For instance, the overall weight value may be assigned based on an analysis at operation 606 indicative of how similar the pixel unit is in chrominance to an expected chrominance of the object, based on an analysis at operation 608 indicative of how similar the pixel unit is in chrominance to an expected chrominance of environmental imagery of the scene, and/or based on additional analyses of local or global characteristics.

In certain implementations, the overall weight value may be assigned based on an a local characteristic analysis associated with operation 606 or 608, another analysis of local characteristics of the pixel unit (e.g., characteristics associated with the chrominance or luminance of the pixel unit, etc.), or a combination of these local characteristic analyses (e.g., based on a combination of the object comparison at operation 606 and the scene comparison at operation 608). As one example of how a local confidence analysis may be translated into a weight value (or one aspect of an overall weight value that combines several such aspects), the chrominance threshold described above in relation to FIG. 7A (and associated with operation 606) will again be considered. In this example, as illustrated in FIG. 7A, apparatus 100 may compare a chrominance characteristic of a particular pixel unit to a chrominance characteristic associated with an extraneous object by 1) determining a distance (e.g., within color space 700), between a first point representative of the chrominance characteristic of the particular pixel unit (e.g., one of points 702) and a second point representative of the chrominance characteristic associated with the object (e.g., point 704), and 2) based on the distance within the color space between the first and second points, assigning a weight value to the particular pixel unit.

In this example, it may be determined that weight value 802 does not exceed lower threshold 806 (and is hence assigned the second weight value) when the distance between the first and second points is greater than a first distance threshold (e.g., when the first point is located outside of an outer radius from the second point) because this large distance indicates that it is very unlikely that the pixel unit depicts the object. Conversely, it may be determined that weight value 802 exceeds upper threshold 804 (and is hence assigned the first weight value) when the distance between the first and second points is less than a second distance threshold (e.g., when the first point is located within an inner radius from the second point) because this small distance indicates that it is highly likely that the pixel unit depicts the object. In other examples, it may be determined that weight value 802 exceeds lower threshold 806 and does not exceed upper threshold 804 (and is hence assigned a particular operative weight value) when the distance between the first and second points is between the first and second distance thresholds (e.g., when the first point is located between the inner and outer radii from the second point). This is because the moderate distance may indicate that the pixel unit is likely to partially depict the object (e.g., some individual pixels of the pixel unit depicting the object and others not depicting the object as a result of the pixel unit being at an edge of the object), or that it is undetermined whether or not the pixel unit depicts the object.

Returning to FIG. 6, a weight value 802 assigned based on operation 606 in this specific example may serve as an overall weight value for the pixel unit or may serve as one factor or aspect that will be accounted for (e.g., combined, averaged, etc.) together with other factors or aspects in a determination of an overall weight value. For example, the weight value 802 assigned based on operation 606 as described above may be combined with one or more separate weight values 802 (e.g., another first, second, or operative weight value) assigned based on similar analyses associated with operation 608 or based on other local characteristics of the pixel unit. Different weight values assigned based on different analyses may be combined in any suitable way. For instance, apparatus 100 may be configured to use, as the overall weight value, the highest weight value returned from any analysis, the lowest weight value returned from any analysis, or a median or mode of all the weight values returned from the analyses. Alternatively, apparatus 100 may be configured to combine the different weight values into an overall weight value by computing a mean average of the weight values. For instance, if the second weight value (e.g., a full weight value of 100%) is returned from operation 606 and the first weight value (e.g., a null weight value of 0%) is returned from operation 608, apparatus 100 may average these two weight values to an operative weight value (e.g., 50%).

Additionally, global analyses such as the object tracking of operation 610 may be used in addition or as an alternative to the local analyses described above. For example, a pixel unit that is completely included within a bounding box for the extraneous object tracked by operation 610 may be assigned a first weight value 802 (e.g., a null weight value), a pixel unit that is completely outside such a bounding box may be assigned a second weight value 802 (e.g., a full weight value), and a pixel unit that is determined to be on (or near) the border of the bounding box may be assigned an operative weight value 802 (e.g., a weight value greater than the first weight value and less than the second weight value). As with weight values 802 described above in relation to the local characteristics, a weight value 802 assigned based on global characteristic analyses in this way may be used as the overall weight value in certain examples, or may comprise one aspect of the overall weight value as it determined by combining a plurality of such aspects. For example, a global weight value determined based on operation 610 may be combined with one or more other global weight values or one or more local weight values in any of the ways described herein (e.g., using a maximum weight value, using a minimum weight value, computing an average weight value, etc.).

At operation 614, one or more unit auto-exposure data points (e.g., a unit auto-exposure value (Vᵢ), a unit auto-exposure target (Tᵢ), etc.) for the current pixel unit (Pᵢ) may be determined. For example, regardless of whether operation 604 reveals a pixel unit to be part of an extraneous object, to not be part of the object, or to be undetermined, operation 614 may analyze characteristics of the pixel unit such as the luminance of the pixel unit to determine how bright the pixel unit is (e.g., the unit auto-exposure value Vᵢ) and/or what brightness value is desirable for the pixel unit (e.g., the unit auto-exposure target Tᵢ). In implementations in which each pixel unit i is implemented by an individual pixel, the unit auto-exposure value and unit auto-exposure target determined at operation 614 may be implemented as a pixel auto-exposure value and a pixel auto-exposure target for the individual pixel. Conversely, in implementations in which each pixel unit i is implemented as a grouping of pixels in a region of the image frame, the unit auto-exposure value and unit auto-exposure target determined at operation 614 may be determined as an average (e.g., mean, median, mode, etc.) of pixel auto-exposure values and/or pixel auto-exposure targets of individual pixels included within the pixel unit. As mentioned, because operation 614 may be independent of operations 604-612, operation 614 may be performed prior, subsequent, or concurrently with operations 604-612.

At operation 616, the output of operation 612 and 614 may be combined to form a weighted pixel unit corresponding to the pixel unit Pᵢ that is being processed. As shown, each weighted pixel unit may include data associated with a unit auto-exposure value (Vᵢ) for the pixel unit Pᵢ, data associated with a unit auto-exposure target (Tᵢ) for the pixel unit Pᵢ, and data associated with a weight value (Wᵢ) for the pixel unit Pᵢ. As will be described in more detail below, weighted pixel units for each pixel unit of an image frame may be used to determine frame auto-exposure data points (e.g., frame auto-exposure values and frame auto-exposure targets) in a manner that discounts the object region associated with the extraneous object. Once all of the pixel units Pᵢ of the image frame (e.g., or a portion thereof, etc.) have been iterated through at operation 602, flow may proceed (Done) to the END of flow diagram 600, at which point operation 504 may be complete and the object region has been identified based on the respective weight values assigned to the pixel units.

Returning to FIG. 5, flow may proceed from operation 504 to operation 510, where apparatus 100 may determine frame auto-exposure data points such as a frame auto-exposure value (V_{F}) for the image frame and a frame auto-exposure target (T_{F}) for the image frame. At operation 510, the frame auto-exposure data points may be determined in a manner that discounts the object region identified at operation 504. For example, the identified object region may be encoded within the weighted pixel units determined during operation 504 (e.g., at operation 616 of FIG. 6) and these weighted pixel units may be used to determine the frame auto-exposure data points.

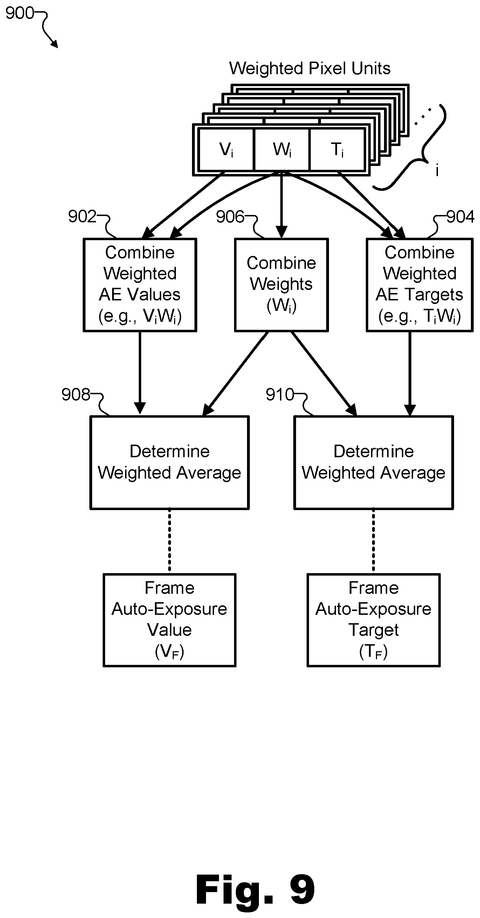

FIG. 9 shows an illustrative flow diagram 900 implementing one way that operation 510 may be performed. Flow diagram 900 shows how apparatus 100 may determine a frame auto-exposure value and a frame auto-exposure target based on weighted pixel units determined as part of the identification of the object region at operation 504. Specifically, as shown, different weighted pixel units (e.g., one for each pixel unit Pᵢ analyzed at operation 504) may provide input data for various operations 902-910 of flow diagram 900 to be performed so that the frame auto-exposure value (V_{F}) and the frame auto-exposure target (T_{F}) are ultimately determined by discounting the object region.

At operation 902, apparatus 100 may scale each unit auto-exposure value Vᵢ from each of the weighted pixel units by the corresponding weight value Wᵢ and may combine (e.g., sum, etc.) these scaled unit auto-exposure values together to form a single value. Similarly, at operation 904, apparatus 100 may scale each unit auto-exposure target Tᵢ from each of the weighted pixel units by the corresponding weight value Wᵢ and may combine (e.g., sum, etc.) these scaled unit auto-exposure targets together to form another single value. At operation 906, apparatus 100 may combine each of the weight values in a similar way (e.g., summing the weight values together or the like).

At operation 908, apparatus 100 may determine the frame auto-exposure value based on the respective weight values assigned to the pixel units. For example, the frame auto-exposure value may be determined as a weighted average of the respective unit auto-exposure values of the pixel units. Apparatus 100 may determine the weighted average at operation 908 based on the output from operations 902 and 906 (e.g., by dividing the output of operation 902 by the output of operation 906) to form the frame auto-exposure value. In this way, the frame auto-exposure value V_{F} may be determined in accordance with Equation 1 (where i is an index used to iterate through each weighted pixel unit): ${V}_{F} = \frac{{\sum }_{i} \left({V}_{i}∗{W}_{i}\right)}{{\sum }_{i} \left({W}_{i}\right)}$ (Eq. 1)

At operation 910, apparatus 100 may determine the frame auto-exposure target based on the respective weight values assigned to the pixel units. For example, the frame auto-exposure target may be determined as a weighted average of the respective unit auto-exposure targets of the pixel units. Apparatus 100 may determine the weighted average at operation 910 based on the output from operations 904 and 906 (e.g., dividing the output of operation 904 by the output of operation 906) to form the frame auto-exposure target. In this way, the frame auto-exposure value T_{F} may be determined in accordance with Equation 2 (where i is an index used to iterate through each weighted pixel unit): ${T}_{F} = \frac{{\sum }_{i} \left({T}_{i}∗{W}_{i}\right)}{{\sum }_{i} \left({W}_{i}\right)}$ (Eq. 2)

In other embodiments, weighted averages incorporating unit auto-exposure data points and weight values for various pixel units may be computed in other ways to similarly discount the identified object region based on the way that pixel units have been weighted to eliminate or downplay the influence on the auto-exposure management of pixel units determined to at least partially correspond to a depiction of an extraneous object within an image frame.

Once a frame auto-exposure value has been determined at operation 908 and a frame auto-exposure target has been determined at operation 910 of FIG. 9, operation 510 of FIG. 5 may be complete and flow may proceed within flow diagram 500 to operation 512, where apparatus 100 may update auto-exposure parameters for the image capture system based on the frame auto-exposure value and/or frame auto-exposure target that have been determined by discounting the object region. At operation 512, apparatus 100 may update (e.g., adjust or maintain) auto-exposure parameters of the image capture system in preparation for the image capture system capturing subsequent image frames in the image frame sequence.

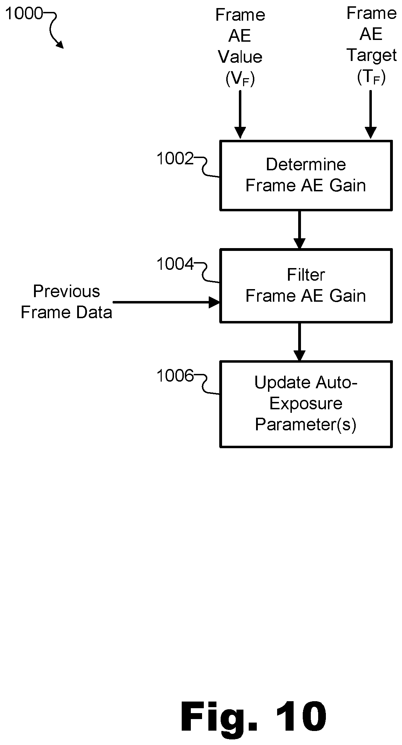

FIG. 10 shows an illustrative technique 1000 for updating an auto-exposure parameter at operation 512. As shown, the frame auto-exposure value and frame auto-exposure target determined previously are used as inputs for operations shown in FIG. 10. For example, an operation 1002 may receive the frame auto-exposure value and frame auto-exposure target as inputs and may use them as a basis for determining a frame auto-exposure gain. The frame auto-exposure gain may be determined to correspond to a ratio of the frame auto-exposure target to the frame auto-exposure value. In this way, if the frame auto-exposure value is already equal to the frame auto-exposure target (e.g., such that no further adjustment is needed to align to the target), the frame auto-exposure gain may be set to a gain of 1, so that the system will neither try to boost nor attenuate the auto-exposure values for a subsequent frame that the image capture system captures. Conversely, if the frame auto-exposure target is different from the frame auto-exposure value, the frame auto-exposure gain may be set to correspond to a value less than or greater than 1 to cause the system to either boost or attenuate the auto-exposure values for the subsequent frame in an attempt to make the auto-exposure values more closely match the desired auto-exposure target.

At operation 1004, the frame auto-exposure gain may be taken as an input along with other data (e.g., other frame auto-exposure gains) determined for previous image frames in the image frame sequence. Based on these inputs, operation 1004 applies filtering to ensure that the auto-exposure gain does not change more quickly than desired and to thereby ensure that image frames presented to the user maintain a consistent brightness and change gradually. The filtering performed at operation 1004 may be performed using a smoothing filter such as a temporal infinite impulse response (IIR) filter or another such digital or analog filter as may serve a particular implementation.

At operation 1006, the filtered frame auto-exposure gain may be used as a basis for adjusting one or more auto-exposure parameters of the image capture system (e.g., for use by the image capture device or the illumination source to capture additional image frames). For example, as described above, adjusted auto-exposure parameters may include an exposure time parameter, a shutter aperture parameter, a luminance gain parameter, or the like. For image capture systems in which the illumination of the scene is largely or completely controlled by the image capture system (e.g., an image capture system including an endoscopic image capture device described above, an image capture system including a flash or other illumination source, etc.), adjusted auto-exposure parameters may further include an illumination intensity parameter, an illumination duration parameter, or the like.

Adjustments to the auto-exposure parameters of the image capture system may cause the image capture system to expose subsequent image frames in various different ways. For example, by adjusting the exposure time parameter, a shutter speed may be adjusted for a shutter included in the image capture system. For instance, the shutter may be held open for a longer period of time (e.g., to thereby increase the amount of exposure time of an image sensor) or for a shorter period of time (e.g., to thereby decrease the amount of exposure time for the image sensor). As another example, by adjusting the shutter aperture parameter, an aperture of the shutter may be adjusted to open more widely (e.g., to thereby increase the amount of light exposed to the image sensor) or less widely (e.g., to thereby decrease the amount of light exposed to the image sensor). As yet another example, by adjusting the luminance gain parameter, a sensitivity (e.g., an ISO sensitivity) may be increased or decreased to amplify or attenuate the illuminance as captured by the image capture system. For implementations in which the image capture system controls the illumination of the scene, the illumination intensity and/or illumination duration parameters may be adjusted to increase the intensity and duration of the light used to illuminate the scene being captured, thereby also affecting how much light the image sensor is exposed to.

Returning to FIG. 5, after the operations of flow diagram 500 have been performed, the current image frame may be considered fully processed by apparatus 100 and flow may return to operation 502, where a subsequent image frame of the image frame sequence may be obtained. The process may be repeated for the subsequent image frame and/or other subsequent image frames. It will be understood that, in certain examples, every image frame may be analyzed in accordance with flow diagram 500 to keep the auto-exposure data points (e.g., frame auto-exposure value and frame auto-exposure target, etc.), and auto-exposure parameters as up-to-date as possible. In other examples, only certain image frames (e.g., every other image frame, every third image frame, etc.) may be so analyzed to conserve processing bandwidth in scenarios where more periodic auto-exposure processing still allows design specifications and targets to be achieved. It will also be understood that auto-exposure effects may tend to lag a few frames behind luminance changes at a scene, since auto-exposure parameter adjustments made based on one particular frame do not affect the exposure of that frame, but rather affect subsequent frames.

Based on any adjustments apparatus 100 makes to the auto-exposure parameters (and/or based on maintaining the auto-exposure parameters at their current levels when appropriate), apparatus 100 may successfully manage auto-exposure for image frames being captured by the image capture system, and subsequent image frames may be captured with desirable auto-exposure properties so as to have an attractive and beneficial appearance when presented to users.

As has been described, apparatus 100, method 200, and/or system 300 may each be associated in certain examples with a computer-assisted medical system used to perform a medical procedure (e.g., a surgical procedure, a diagnostic procedure, an exploratory procedure, etc.) on a body. To illustrate, FIG. 11 shows an illustrative computer-assisted medical system 1100 that may be used to perform various types of medical procedures including surgical and/or non-surgical procedures.

As shown, computer-assisted medical system 1100 may include a manipulator assembly 1102 (a manipulator cart is shown in FIG. 11), a user control apparatus 1104, and an auxiliary apparatus 1106, all of which are communicatively coupled to each other. Computer-assisted medical system 1100 may be utilized by a medical team to perform a computer-assisted medical procedure or other similar operation on a body of a patient 1108 or on any other body as may serve a particular implementation. As shown, the medical team may include a first user 1110-1 (such as a surgeon for a surgical procedure), a second user 1110-2 (such as a patient-side assistant), a third user 1110-3 (such as another assistant, a nurse, a trainee, etc.), and a fourth user 1110-4 (such as an anesthesiologist for a surgical procedure), all of whom may be collectively referred to as users 1110, and each of whom may control, interact with, or otherwise be a user of computer-assisted medical system 1100. More, fewer, or alternative users may be present during a medical procedure as may serve a particular implementation. For example, team composition for different medical procedures, or for non-medical procedures, may differ and include users with different roles.

While FIG. 11 illustrates an ongoing minimally invasive medical procedure such as a minimally invasive surgical procedure, it will be understood that computer-assisted medical system 1100 may similarly be used to perform open medical procedures or other types of operations. For example, operations such as exploratory imaging operations, mock medical procedures used for training purposes, and/or other operations may also be performed.

As shown in FIG. 11, manipulator assembly 1102 may include one or more manipulator arms 1112 (e.g., manipulator arms 1112-1 through 1112-4) to which one or more instruments may be coupled. The instruments may be used for a computer-assisted medical procedure on patient 1108 (e.g., in a surgical example, by being at least partially inserted into patient 1108 and manipulated within patient 1108). While manipulator assembly 1102 is depicted and described herein as including four manipulator arms 1112, it will be recognized that manipulator assembly 1102 may include a single manipulator arm 1112 or any other number of manipulator arms as may serve a particular implementation. While the example of FIG. 11 illustrates manipulator arms 1112 as being robotic manipulator arms, it will be understood that, in some examples, one or more instruments may be partially or entirely manually controlled, such as by being handheld and controlled manually by a person. For instance, these partially or entirely manually controlled instruments may be used in conjunction with, or as an alternative to, computer-assisted instrumentation that is coupled to manipulator arms 1112 shown in FIG. 11.

During the medical operation, user control apparatus 1104 may be configured to facilitate teleoperational control by user 1110-1 of manipulator arms 1112 and instruments attached to manipulator arms 1112. To this end, user control apparatus 1104 may provide user 1110-1 with imagery of an operational area associated with patient 1108 as captured by an imaging device. To facilitate control of instruments, user control apparatus 1104 may include a set of master controls. These master controls may be manipulated by user 1110-1 to control movement of the manipulator arms 1112 or any instruments coupled to manipulator arms 1112.

Auxiliary apparatus 1106 may include one or more computing devices configured to perform auxiliary functions in support of the medical procedure, such as providing insufflation, electrocautery energy, illumination or other energy for imaging devices, image processing, or coordinating components of computer-assisted medical system 1100. In some examples, auxiliary apparatus 1106 may be configured with a display monitor 1114 configured to display one or more user interfaces, or graphical or textual information in support of the medical procedure. In some instances, display monitor 1114 may be implemented by a touchscreen display and provide user input functionality.

As will be described in more detail below, apparatus 100 may be implemented within or may operate in conjunction with computer-assisted medical system 1100. For instance, in certain implementations, apparatus 100 may be implemented by computing resources included within an instrument (e.g., an endoscopic or other imaging instrument) attached to one of manipulator arms 1112, or by computing resources associated with manipulator assembly 1102, user control apparatus 1104, auxiliary apparatus 1106, or another system component not explicitly shown in FIG. 11.

Manipulator assembly 1102, user control apparatus 1104, and auxiliary apparatus 1106 may be communicatively coupled one to another in any suitable manner. For example, as shown in FIG. 11, manipulator assembly 1102, user control apparatus 1104, and auxiliary apparatus 1106 may be communicatively coupled by way of control lines 1116, which may represent any wired or wireless communication link as may serve a particular implementation. To this end, manipulator assembly 1102, user control apparatus 1104, and auxiliary apparatus 1106 may each include one or more wired or wireless communication interfaces, such as one or more local area network interfaces, Wi-Fi network interfaces, cellular interfaces, and so forth.

In certain embodiments, one or more of the processes described herein may be implemented at least in part as instructions embodied in a non-transitory computer-readable medium and executable by one or more computing devices. In general, a processor (e.g., a microprocessor) receives instructions, from a non-transitory computer-readable medium, (e.g., a memory, etc.), and executes those instructions, thereby performing one or more processes, including one or more of the processes described herein. Such instructions may be stored and/or transmitted using any of a variety of known computer-readable media.

A computer-readable medium (also referred to as a processor-readable medium) includes any non-transitory medium that participates in providing data (e.g., instructions) that may be read by a computer (e.g., by a processor of a computer). Such a medium may take many forms, including, but not limited to, non-volatile media, and/or volatile media. Non-volatile media may include, for example, optical or magnetic disks and other persistent memory. Volatile media may include, for example, dynamic random access memory (DRAM), which typically constitutes a main memory. Common forms of computer-readable media include, for example, a disk, hard disk, magnetic tape, any other magnetic medium, a compact disc read-only memory (CD-ROM), a digital video disc (DVD), any other optical medium, random access memory (RAM), programmable read-only memory (PROM), electrically erasable programmable read-only memory (EPROM), FLASH-EEPROM, any other memory chip or cartridge, or any other tangible medium from which a computer can read.

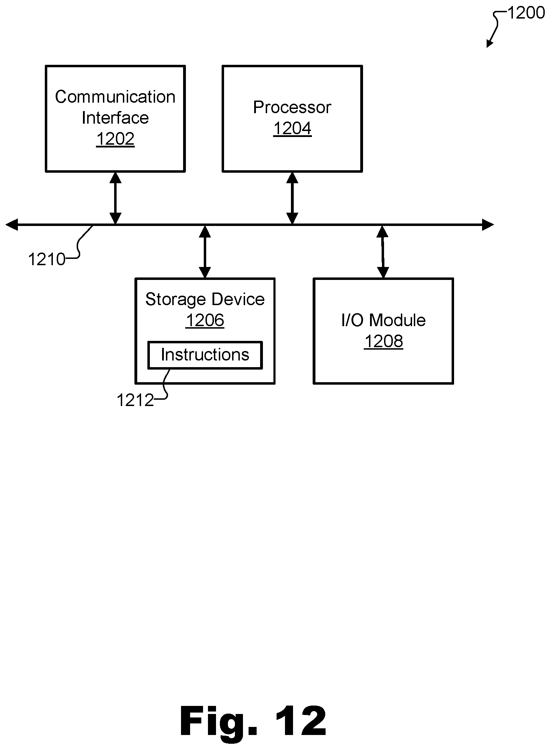

FIG. 12 shows an illustrative computing system 1200 that may be specifically configured to perform one or more of the processes described herein. For example, computing system 1200 may include or implement (or partially implement) an auto-exposure management apparatus such as apparatus 100, an auto-exposure management system such as system 300, or any other computing systems or devices described herein.

As shown in FIG. 12, computing system 1200 may include a communication interface 1202, a processor 1204, a storage device 1206, and an input/output ("I/O") module 1208 communicatively connected via a communication infrastructure 1210. While an illustrative computing system 1200 is shown in FIG. 12, the components illustrated in FIG. 12 are not intended to be limiting. Additional or alternative components may be used in other embodiments. Components of computing system 1200 shown in FIG. 12 will now be described in additional detail.

Communication interface 1202 may be configured to communicate with one or more computing devices. Examples of communication interface 1202 include, without limitation, a wired network interface (such as a network interface card), a wireless network interface (such as a wireless network interface card), a modem, an audio/video connection, and any other suitable interface.

Processor 1204 generally represents any type or form of processing unit capable of processing data or interpreting, executing, and/or directing execution of one or more of the instructions, processes, and/or operations described herein. Processor 1204 may direct execution of operations in accordance with one or more applications 1212 or other computer-executable instructions such as may be stored in storage device 1206 or another computer-readable medium.

Storage device 1206 may include one or more data storage media, devices, or configurations and may employ any type, form, and combination of data storage media and/or device. For example, storage device 1206 may include, but is not limited to, a hard drive, network drive, flash drive, magnetic disc, optical disc, RAM, dynamic RAM, other non-volatile and/or volatile data storage units, or a combination or subcombination thereof. Electronic data, including data described herein, may be temporarily and/or permanently stored in storage device 1206. For example, data representative of one or more executable applications 1212 configured to direct processor 1204 to perform any of the operations described herein may be stored within storage device 1206. In some examples, data may be arranged in one or more databases residing within storage device 1206.

I/O module 1208 may include one or more I/O modules configured to receive user input and provide user output. One or more I/O modules may be used to receive input for a single virtual experience. I/O module 1208 may include any hardware, firmware, software, or combination thereof supportive of input and output capabilities. For example, I/O module 1208 may include hardware and/or software for capturing user input, including, but not limited to, a keyboard or keypad, a touchscreen component (e.g., touchscreen display), a receiver (e.g., an RF or infrared receiver), motion sensors, and/or one or more input buttons.

I/O module 1208 may include one or more devices for presenting output to a user, including, but not limited to, a graphics engine, a display (e.g., a display screen), one or more output drivers (e.g., display drivers), one or more audio speakers, and one or more audio drivers. In certain embodiments, I/O module 1208 is configured to provide graphical data to a display for presentation to a user. The graphical data may be representative of one or more graphical user interfaces and/or any other graphical content as may serve a particular implementation.

In some examples, any of the facilities described herein may be implemented by or within one or more components of computing system 1200. For example, one or more applications 1212 residing within storage device 1206 may be configured to direct processor 1204 to perform one or more processes or functions associated with processor 104 of apparatus 100. Likewise, memory 102 of apparatus 100 may be implemented by or within storage device 1206.

In the preceding description, various illustrative embodiments have been described with reference to the accompanying drawings. It will, however, be evident that various modifications and changes may be made thereto, and additional embodiments may be implemented, without departing from the scope of the invention as set forth in the claims that follow. For example, certain features of one embodiment described herein may be combined with or substituted for features of another embodiment described herein. The description and drawings are accordingly to be regarded in an illustrative rather than a restrictive sense.

## Claims

1. An apparatus (100) comprising:
one or more processors (104); and
memory (102) storing executable instructions (106) that, when executed by the one or more processors (104), cause the apparatus (100) to:
identify, in an image frame (402) captured by an image capture system, a plurality of pixel units corresponding to a depiction of an object (406) portrayed in the image frame (402);
determine, for each of the plurality of pixel units, a confidence level that the respective pixel unit corresponds to the depiction of the object (406);
determine a frame auto-exposure value for the image frame (402) by discounting the influence of the plurality of pixel units on the frame auto-exposure value, wherein the discounting is performed by weighting the plurality of pixel units based on the confidence levels of the plurality of pixel units such that contributions of pixel units of the plurality of pixel units are discounted to different extents based on different weight values derived from the confidence levels of the pixel units; and
update, based on the frame auto-exposure value, one or more auto-exposure parameters for use by the image capture system to capture an additional image frame.

2. The apparatus (100) of claim 1, wherein:
the identifying the plurality of pixel units corresponding to the depiction of the object (406) is based on a difference in colour of the object (406) and colour of body tissue depicted in the image frame (402).

3. The apparatus (100) of claim 1 or 2, wherein:
the identifying the plurality of pixel units corresponding to the depiction of the object (406) is based on one or more local characteristics associated with pixel units included in the image frame (402);
the one or more local characteristics associated with the pixel units included in the image frame (402) include chrominance characteristics of the pixel units included in the image frame (402); and
the identifying the plurality of pixel units corresponding to the depiction of the object (406) includes comparing the chrominance characteristics of the pixel units included in the image frame (402) to a chrominance characteristic associated with the object (406).

4. The apparatus (100) of claim 3, wherein:
the comparing the chrominance characteristics of the pixel units included in the image frame (402) to the chrominance characteristic associated with the object (406) includes, for each individual pixel unit of the pixel units included in the image frame (402):
determining a distance, within a color space (700), between a first point representative of a chrominance characteristic of the individual pixel unit and a second point representative of the chrominance characteristic associated with the object (406);
determining, based on the distance, the confidence level for the individual pixel unit; and
based on the confidence level for the individual pixel unit, assigning a weight value (802) to the individual pixel unit, the weight value assigned as:
a first weight value if the confidence level exceeds an upper threshold (804),
a second weight value if the confidence level does not exceed a lower threshold (806), wherein the second weight value is greater than the first weight value, and
an operative weight value between the first weight value and the second weight value if the confidence level exceeds the lower threshold (806) and does not exceed the upper threshold (804); and
the identifying the plurality of pixel units corresponding to the depiction of the object (406)( is performed based on the weight value 802 assigned to the individual pixel unit.

5. The apparatus (100) of any of claims 1-2, wherein:
the identifying the plurality of pixel units corresponding to the depiction of the object (406) is based on one or more local characteristics associated with pixel units included in the image frame (402);
the one or more local characteristics associated with the pixel units included in the image frame (402) include chrominance and luminance characteristics of the pixel units included in the image frame (402); and
the identifying the plurality of pixel units corresponding to the depiction of the object (406) includes:
comparing the chrominance characteristics of the pixel units included in the image frame (402) to a chrominance characteristic associated with the object (406), and
comparing the luminance characteristics of the pixel units included in the image frame (402) to a luminance characteristic associated with the object (406).

6. The apparatus (100) of any of claims 1-2, wherein:
the instructions (106), when executed by the one or more processors (104), cause the apparatus (100) to determine a color gamut for environmental imagery of a scene depicted in the image frame (402);
the identifying the plurality of pixel units corresponding to the depiction of the object (406) is based on one or more local characteristics associated with pixel units included in the image frame (402);
the one or more local characteristics associated with the pixel units included in the image frame (402) include chrominance characteristics of the pixel units included in the image frame (402); and
the identifying the plurality of pixel units corresponding to the depiction of the object (406) includes determining whether the chrominance characteristics of the pixel units included in the image frame (402) are included within the color gamut for the environmental imagery of the scene.

7. The apparatus (100) of claim 6, wherein:
the image capture system includes an endoscopic image capture device configured to capture the image frame (402) as part of an image frame sequence captured during a performance of a medical procedure on a body;
the scene depicted in the image frame (402) is an internal view of the body; and
the color gamut for the environmental imagery of the scene encompasses a range of red colors corresponding to blood and tissue visible in the internal view of the body.

8. The apparatus (100) of any of claims 1-7, wherein:
the identifying the plurality of pixel units corresponding to the depiction of the object (406) is based on an object position characteristic determined based on object tracking data received from an object tracking system that tracks a position of the object (406) within a scene depicted in the image frame (402).

9. The apparatus (100) of claim 8, wherein:
the object (406) comprises an instrument controlled by a robotic arm; and
the object tracking system tracks the position of the object (406) based on kinematic data associated with movements of the robotic arm.

10. The apparatus (100) of claim 8, wherein the object tracking system tracks the position of the object (406) based on computer vision techniques applied to image frames of an image frame sequence that includes the image frame (402).

11. The apparatus (100) of any of claims 1-10, wherein the identifying the plurality of pixel units corresponding to the depiction of the object is based on a combination of:
a chrominance or luminance characteristic associated with a pixel unit included in the image frame (402); and
a position characteristic of the object (406).

12. A method (200) comprising:
identifying, by a computing device and in an image frame (402) captured by an image capture system, a plurality of pixel units corresponding to a depiction of an object (406) portrayed in the image frame (402);
determining, by the computing device and for each of the plurality of pixel units, a confidence level that the respective pixel unit corresponds to the depiction of the object (406);
determining, by the computing device, a frame auto-exposure value and a frame auto-exposure target for the image frame by discounting the influence of the plurality of pixel units, wherein the discounting is performed by weighting the plurality of pixel units based on the confidence levels of the plurality of pixel units such that contributions of pixel units of the plurality of pixel units are discounted to different extents based on different weight values derived from the confidence levels of the pixel units; and
updating, by the computing device and based on the frame auto-exposure value and the frame auto-exposure target, one or more auto-exposure parameters for use by the image capture system to capture an additional image frame.

13. The method (200) of claim 12, wherein:
the identifying the plurality of pixel units corresponding to the depiction of the object (406) is based on one or more local characteristics associated with pixel units included in the image frame (402);
the one or more local characteristics associated with the pixel units included in the image frame (402) include chrominance characteristics of the pixel units included in the image frame (402); and
the identifying the plurality of pixel units corresponding to the depiction of the object (406)includes comparing the chrominance characteristics of the pixel units included in the image frame (402) to a chrominance characteristic associated with the object (406).

14. The method (402) of claim 13, wherein:
the comparing the chrominance characteristics of the pixel units included in the image frame (402) to the chrominance characteristic associated with the object (406) includes, for each individual pixel unit of the pixel units included in the image frame (402):
determining a distance, within a color space (700), between a first point representative of a chrominance characteristic of the individual pixel unit and a second point representative of the chrominance characteristic associated with the object; determining, based on the distance, the confidence level for the individual pixel unit; and
based on the confidence level for the individual pixel unit, assigning a weight value (802) to the individual pixel unit, the weight value (802) assigned as:
a first weight value if the confidence level exceeds an upper threshold (804),
a second weight value if the confidence level does not exceed a lower threshold (806), wherein the second weight value is greater than the first weight value, and
an operative weight value between the first weight value and the second weight value if the confidence level exceeds the lower threshold (806) and does not exceed the upper threshold (804); and
the identifying the plurality of pixel units corresponding to the depiction of the object is performed based on the weight value (802) assigned to the individual pixel unit.

15. The method (200) of any of claims 12-14, wherein the identifying the plurality of pixel units corresponding to the depiction of the object (406) is based on a combination of:
a chrominance characteristic associated with a pixel unit included in the image frame (402); and
a position characteristic of the object (406).

## Patentansprüche

1. Gerät (100), umfassend:
einen oder mehrere Prozessoren (104); und
einen Speicher (102), der ausführbare Anweisungen (106) speichert, die bei Ausführung durch den einen oder die mehreren Prozessoren (104) das Gerät (100) veranlassen zum:
Identifizieren einer Vielzahl von Pixeleinheiten, die einer Darstellung eines Objekts (406) entspricht, das in einem Bildrahmen (402) porträtiert ist, in dem Bildrahmen (402), der von einem Bildaufnahmesystem aufgenommen wird;
für jede von der Vielzahl von Pixeleinheiten Bestimmen eines Konfidenzniveaus, dass die jeweilige Pixeleinheit der Darstellung des Objekts (406) entspricht;
Bestimmen eines Rahmenautobelichtungswerts für den Bildrahmen (402) durch Diskontieren des Einflusses der Vielzahl von Pixeleinheiten auf den Rahmenautobelichtungswert, wobei das Diskontieren durch Gewichten der Vielzahl von Pixeleinheiten auf der Basis der Konfidenzniveaus der Vielzahl von Pixeleinheiten durchgeführt wird, so dass Beiträge von Pixeleinheiten von der Vielzahl von Pixeleinheiten auf der Basis von unterschiedlichen Gewichtswerten, die von den Konfidenzniveaus der Pixeleinheiten abgeleitet sind, in unterschiedlichem Maße diskontiert werden; und
Aktualisieren eines oder mehrerer Autobelichtungsparameter zur Verwendung durch das Bildaufnahmesystem auf der Basis des Rahmenautobelichtungswerts, um einen zusätzlichen Bildrahmen aufzunehmen.

2. Gerät (100) nach Anspruch 1, wobei:
das Identifizieren der Vielzahl von Pixeleinheiten, die der Darstellung des Objekts (406) entspricht, auf einem Unterschied einer Farbe des Objekts (406) und einer Farbe eines Körpergewebes basiert, das in dem Bildrahmen (402) dargestellt wird.

3. Gerät (100) nach Anspruch 1 oder 2, wobei:
das Identifizieren der Vielzahl von Pixeleinheiten, die der Darstellung des Objekts (406) entspricht, auf einem oder mehreren lokalen Charakteristika basiert, die mit Pixeleinheiten assoziiert sind, die in dem Bildrahmen (402) eingeschlossen sind;
das eine oder die mehreren lokalen Charakteristika, die mit den Pixeleinheiten assoziiert sind, die in dem Bildrahmen (402) eingeschlossen sind, Chrominanzcharakteristika der Pixeleinheiten einschließen, die in dem Bildrahmen (402) eingeschlossen sind; und
das Identifizieren der Vielzahl von Pixeleinheiten, die der Darstellung des Objekts (406) entspricht, ein Vergleichen der Chrominanzcharakteristika der Pixeleinheiten, die in dem Bildrahmen (402) eingeschlossen sind, mit einem Chrominanzcharakteristikum einschließt, das mit dem Objekt (406) assoziiert ist.

4. Gerät (100) nach Anspruch 3, wobei:
das Vergleichen der Chrominanzcharakteristika der Pixeleinheiten, die in dem Bildrahmen (402) eingeschlossen sind, mit dem Chrominanzcharakteristikum, das mit dem Objekt (406) assoziiert ist, für jede einzelne Pixeleinheit von den Pixeleinheiten, die in dem Bildrahmen (402) eingeschlossen sind, einschließt:
Bestimmen eines Abstands zwischen einem ersten Punkt, der ein Chrominanzcharakteristikum der einzelnen Pixeleinheit repräsentiert, und einem zweiten Punkt, der das Chrominanzcharakteristikum repräsentiert, das mit dem Objekt (406) assoziiert ist, innerhalb eines Farbraums (700);
Bestimmen des Konfidenzniveaus für die einzelne Pixeleinheit auf der Basis des Abstands; und
Zuteilen eines Gewichtswerts (802) zu der einzelnen Pixeleinheit auf der Basis des Konfidenzniveaus für die einzelne Pixeleinheit, wobei der Gewichtswert zugeteilt wird als:
ein erster Gewichtswert, wenn das Konfidenzniveau einen oberen Schwellenwert (804) übersteigt,
ein zweiter Gewichtswert, wenn das Konfidenzniveau einen unteren Schwellenwert (806) nicht übersteigt, wobei der zweite Gewichtswert größer als der erste Gewichtswert ist, und
ein operativer Gewichtswert zwischen dem ersten Gewichtswert und dem zweiten Gewichtswert, wenn das Konfidenzniveau den unteren Schwellenwert (806) übersteigt und den oberen Schwellenwert (804) nicht übersteigt; und
das Identifizieren der Vielzahl von Pixeleinheiten, die der Darstellung des Objekts (406) entspricht, auf der Basis des Gewichtswerts 802 durchgeführt wird, der der einzelnen Pixeleinheit zugeteilt wird.

5. Gerät (100) nach einem der Ansprüche 1-2, wobei:
das Identifizieren der Vielzahl von Pixeleinheiten, die der Darstellung des Objekts (406) entspricht, auf einem oder mehreren lokalen Charakteristika basiert, die mit Pixeleinheiten assoziiert sind, die in dem Bildrahmen (402) eingeschlossen sind;
das eine oder die mehreren lokalen Charakteristika, die mit den Pixeleinheiten assoziiert sind, die in dem Bildrahmen (402) eingeschlossen sind, Chrominanz- und Luminanzcharakteristika der Pixeleinheiten einschließen, die in dem Bildrahmen (402) eingeschlossen sind; und
das Identifizieren der Vielzahl von Pixeleinheiten, die der Darstellung des Objekts (406) entspricht, einschließt:
Vergleichen der Chrominanzcharakteristika der Pixeleinheiten, die in dem Bildrahmen (402) eingeschlossen sind, mit einem Chrominanzcharakteristikum, das mit dem Objekt (406) assoziiert ist, und
Vergleichen der Luminanzcharakteristika der Pixeleinheiten, die in dem Bildrahmen (402) eingeschlossen sind, mit einem Luminanzcharakteristikum, das mit dem Objekt (406) assoziiert ist.

6. Gerät (100) nach einem der Ansprüche 1-2, wobei:
die Anweisungen (106) bei Ausführung durch den einen oder die mehreren Prozessoren (104) das Gerät (100) dazu veranlassen, eine Farbskala für Umgebungsbildmaterial einer Szene zu bestimmen, die in dem Bildrahmen (402) dargestellt ist;
das Identifizieren der Vielzahl von Pixeleinheiten, die der Darstellung des Objekts (406) entspricht, auf einem oder mehreren lokalen Charakteristika basiert, die mit Pixeleinheiten assoziiert sind, die in dem Bildrahmen (402) eingeschlossen sind;
das eine oder die mehreren lokalen Charakteristika, die mit den Pixeleinheiten assoziiert sind, die in dem Bildrahmen (402) eingeschlossen sind, Chrominanzcharakteristika der Pixeleinheiten einschließen, die in dem Bildrahmen (402) eingeschlossen sind; und
das Identifizieren der Vielzahl von Pixeleinheiten, die der Darstellung des Objekts (406) entspricht, ein Bestimmen einschließt, ob die Chrominanzcharakteristika der Pixeleinheiten, die in dem Bildrahmen (402) eingeschlossen sind, innerhalb der Farbskala des Umgebungsbildmaterials der Szene eingeschlossen sind.

7. Gerät (100) nach Anspruch 6, wobei:
das Bildaufnahmesystem eine endoskopische Bildaufnahmevorrichtung einschließt, die dazu konfiguriert ist, den Bildrahmen (402) als Teil einer Bildrahmensequenz aufzunehmen, die während einer Durchführung eines medizinischen Eingriffs an einem Körper aufgenommen wird;
die Szene, die in dem Bildrahmen (402) dargestellt ist, eine Innenansicht des Körpers ist; und
die Farbskala für das Umgebungsbildmaterial der Szene ein Sortiment von roten Farben beinhaltet, die Blut und Gewebe entsprechen, die in der Innenansicht des Körpers sichtbar sind.

8. Gerät (100) nach einem der Ansprüche 1-7, wobei:
das Identifizieren der Vielzahl von Pixeleinheiten, die der Darstellung des Objekts (406) entspricht, auf einem Objektpositionscharakteristikum basiert, das auf der Basis von Objektverfolgungsdaten bestimmt wird, die von einem Objektverfolgungssystem erhalten werden, das eine Position des Objekts (406) innerhalb einer Szene verfolgt, die in dem Bildrahmen (402) dargestellt ist.

9. Gerät (100) nach Anspruch 8, wobei:
das Objekt (406) ein Instrument umfasst, das durch einen Roboterarm gesteuert wird; und
das Objektverfolgungssystem die Position des Objekts (406) auf der Basis von kinematischen Daten verfolgt, die mit Bewegungen des Roboterarms assoziiert sind.

10. Gerät (100) nach Anspruch 8, wobei das Objektverfolgungssystem die Position des Objekts (406) auf der Basis von Computer-Vision-Techniken verfolgt, die auf Bildrahmen einer Bildrahmensequenz angewendet werden, die den Bildrahmen (402) einschließt.

11. Gerät (100) nach einem der Ansprüche 1-10, wobei das Identifizieren der Vielzahl von Pixeleinheiten, die der Darstellung des Objekts entspricht, basiert auf einer Kombination von:
einem Chrominanz- oder Luminanzcharakteristikum, das mit einer Pixeleinheit assoziiert ist, die in dem Bildrahmen (402) eingeschlossen ist; und
einem Positionscharakteristikum des Objekts (406).

12. Verfahren (200), umfassend:
Identifizieren einer Vielzahl von Pixeleinheiten, die einer Darstellung eines Objekts (406) entspricht, das in einem Bildrahmen (402) porträtiert ist, durch eine Rechenvorrichtung und in dem Bildrahmen (402), der von einem Bildaufnahmesystem aufgenommen wird;
durch die Rechenvorrichtung und für jede von der Vielzahl von Pixeleinheiten Bestimmen eines Konfidenzniveaus, dass die jeweilige Pixeleinheit der Darstellung des Objekts (406) entspricht;
Bestimmen eines Rahmenautobelichtungswerts und eines Rahmenautobelichtungsziels für den Bildrahmen durch Diskontieren des Einflusses der Vielzahl von Pixeleinheiten durch die Rechenvorrichtung, wobei das Diskontieren durch Gewichten der Vielzahl von Pixeleinheiten auf der Basis der Konfidenzniveaus der Vielzahl von Pixeleinheiten durchgeführt wird, so dass Beiträge von Pixeleinheiten von der Vielzahl von Pixeleinheiten auf der Basis von unterschiedlichen Gewichtswerten, die von den Konfidenzniveaus der Pixeleinheiten abgeleitet sind, in unterschiedlichem Maße diskontiert werden; und
Aktualisieren eines oder mehrerer Autobelichtungsparameter zur Verwendung durch das Bildaufnahmesystem durch die Rechenvorrichtung und auf der Basis des Rahmenautobelichtungswerts und des Rahmenautobelichtungsziels, um einen zusätzlichen Bildrahmen aufzunehmen.

13. Verfahren (200) nach Anspruch 12, wobei:
das Identifizieren der Vielzahl von Pixeleinheiten, die der Darstellung des Objekts (406) entspricht, auf einem oder mehreren lokalen Charakteristika basiert, die mit Pixeleinheiten assoziiert sind, die in dem Bildrahmen (402) eingeschlossen sind;
das eine oder die mehreren lokalen Charakteristika, die mit den Pixeleinheiten assoziiert sind, die in dem Bildrahmen (402) eingeschlossen sind, Chrominanzcharakteristika der Pixeleinheiten einschließen, die in dem Bildrahmen (402) eingeschlossen sind; und
das Identifizieren der Vielzahl von Pixeleinheiten, die der Darstellung des Objekts (406) entspricht, ein Vergleichen der Chrominanzcharakteristika der Pixeleinheiten, die in dem Bildrahmen (402) eingeschlossen sind, mit einem Chrominanzcharakteristikum einschließt, das mit dem Objekt (406) assoziiert ist.

14. Verfahren (402) nach Anspruch 13, wobei:
das Vergleichen der Chrominanzcharakteristika der Pixeleinheiten, die in dem Bildrahmen (402) eingeschlossen sind, mit dem Chrominanzcharakteristikum, das mit dem Objekt (406) assoziiert ist, für jede einzelne Pixeleinheit von den Pixeleinheiten, die in dem Bildrahmen (402) eingeschlossen sind, einschließt:
Bestimmen eines Abstands zwischen einem ersten Punkt, der ein Chrominanzcharakteristikum der einzelnen Pixeleinheit repräsentiert, und einem zweiten Punkt, der das Chrominanzcharakteristikum repräsentiert, das mit dem Objekt assoziiert ist, innerhalb eines Farbraums (700);
Bestimmen des Konfidenzniveaus für die einzelne Pixeleinheit auf der Basis des Abstands; und
Zuteilen eines Gewichtswerts (802) zu der einzelnen Pixeleinheit auf der Basis des Konfidenzniveaus für die einzelne Pixeleinheit, wobei der Gewichtswert (802) zugeteilt wird als:
ein erster Gewichtswert, wenn das Konfidenzniveau einen oberen Schwellenwert (804) übersteigt,
ein zweiter Gewichtswert, wenn das Konfidenzniveau einen unteren Schwellenwert (806) nicht übersteigt, wobei der zweite Gewichtswert größer als der erste Gewichtswert ist, und
ein operativer Gewichtswert zwischen dem ersten Gewichtswert und dem zweiten Gewichtswert, wenn das Konfidenzniveau den unteren Schwellenwert (806) übersteigt und den oberen Schwellenwert (804) nicht übersteigt; und
das Identifizieren der Vielzahl von Pixeleinheiten, die der Darstellung des Objekts entspricht, auf der Basis des Gewichtswerts (802) durchgeführt wird, der der einzelnen Pixeleinheit zugeteilt wird.

15. Verfahren (200) nach einem der Ansprüche 12-14, wobei das Identifizieren der Vielzahl von Pixeleinheiten, die der Darstellung des Objekts (406) entspricht, basiert auf einer Kombination von:
einem Chrominanzcharakteristikum, das mit einer Pixeleinheit assoziiert ist, die in dem Bildrahmen (402) eingeschlossen ist; und
einem Positionscharakteristikum des Objekts (406).

## Revendications

1. Appareil (100) comprenant :
un ou plusieurs processeurs (104) ; et
une mémoire (102) stockant des instructions exécutables (106) qui, lorsqu'elles sont exécutées par les un ou plusieurs processeurs (104), amènent l'appareil (100) à :
identifier, dans un cadre d'image (402) capturé par un système de capture d'image, une pluralité d'unités de pixels correspondant à une représentation d'un objet (406) illustré dans le cadre d'image (402) ;
déterminer, pour chacune de la pluralité d'unités de pixels, un niveau de confiance que l'unité de pixel respective correspond à la représentation de l'objet (406) ;
déterminer une valeur d'auto-exposition de cadre pour le cadre d'image (402) en écartant l'influence de la pluralité d'unités de pixels sur la valeur d'auto-exposition de cadre dans lequel l'écartement est effectué en pondérant la pluralité d'unités de pixels sur la base des niveaux de confiance de la pluralité d'unités de pixels de sorte que des contributions d'unités de pixels de la pluralité d'unités de pixels sont écartées dans différentes mesures sur la base de valeurs de pondération différentes dérivées des niveaux de confiance des unités de pixels ; et
mettre à jour, sur la base de la valeur d'auto-exposition de cadre, un ou de plusieurs paramètres d'auto-exposition destinés à être utilisés par le système de capture d'image pour capturer un cadre d'image supplémentaire.

2. Appareil (100) selon la revendication 1, dans lequel :
l'identification de la pluralité d'unités de pixels correspondant à la représentation de l'objet (406) est basée sur une différence de la couleur de l'objet (406) et de la couleur du tissu corporel représenté dans le cadre d'image (402).

3. Appareil (100) selon la revendication 1 ou 2, dans lequel :
l'identification de la pluralité d'unités de pixels correspondant à la représentation de l'objet (406) est basée sur une ou plusieurs caractéristiques locales associées aux unités de pixels incluses dans le cadre d'image (402) ;
les une ou plusieurs caractéristiques locales associées aux unités de pixels incluses dans le cadre d'image (402) incluent des caractéristiques de chrominance des unités de pixels incluses dans le cadre d'image (402) ; et
l'identification de la pluralité d'unités de pixels correspondant à la représentation de l'objet (406) inclut la comparaison des caractéristiques de chrominance des unités de pixels incluses dans le cadre d'image (402) avec une caractéristique de chrominance associée à l'objet (406).

4. Appareil (100) selon la revendication 3, dans lequel :
la comparaison des caractéristiques de chrominance des unités de pixels incluses dans le cadre d'image (402) avec les caractéristiques de chrominance associées à l'objet (406) inclut, pour chaque unité de pixel individuelle des unités de pixels incluses dans le cadre d'image (402) :
la détermination d'une distance, au sein d'un espace de couleur (700), entre un premier point représentatif d'une caractéristique de chrominance de l'unité de pixel individuelle et un deuxième point représentatif de la caractéristique de chrominance associée à l'objet (406) ;
la détermination, sur la base de la distance, du niveau de confiance pour l'unité de pixel individuelle ; et sur la base du niveau de confiance pour l'unité de pixel individuelle, l'attribution d'une valeur de pondération (802) à l'unité de pixel individuelle, la valeur de pondération attribuée comme :
une première valeur de pondération si le niveau de confiance dépasse un seuil supérieur (804),
une deuxième valeur de pondération si le niveau de confiance ne dépasse pas un seuil inférieur (806), dans lequel la deuxième valeur de pondération est supérieure à la première valeur de pondération, et
une valeur de pondération opérationnelle entre la première valeur de pondération et la deuxième valeur de pondération si le niveau de confiance dépasse le seuil inférieur (806) et ne dépasse pas le seuil supérieur (804) ; et
l'identification de la pluralité d'unités de pixels correspondant à la représentation de l'objet (406) est réalisée sur la base de la valeur de pondération 802 attribuée à l'unité de pixel individuelle.

5. Appareil (100) selon l'une quelconque des revendications 1 à 2, dans lequel :
l'identification de la pluralité d'unités de pixels correspondant à la représentation de l'objet (406) est basée sur une ou plusieurs caractéristiques locales associées aux unités de pixels incluses dans le cadre d'image (402) ;
les une ou plusieurs caractéristiques locales associées aux unités de pixels incluses dans le cadre d'image (402) incluent des caractéristiques de chrominance et de luminance des unités de pixels incluses dans le cadre d'image (402) ; et
l'identification de la pluralité d'unités de pixels correspondant à la représentation de l'objet (406) inclut :
la comparaison des caractéristiques de chrominance des unités de pixels incluses dans le cadre d'image (402) avec une caractéristique de chrominance associée à l'objet (406), et
la comparaison des caractéristiques de luminance des unités de pixels incluses dans le cadre d'image (402) avec une caractéristique de luminance associée à l'objet (406).

6. Appareil (100) selon l'une quelconque des revendications 1 à 2, dans lequel :
les instructions (106), quand elles sont exécutées par les un ou plusieurs processeurs (104), amènent l'appareil (100) à déterminer une palette de couleurs pour une imagerie environnementale d'une scène représentée dans le cadre d'image (402) ;
l'identification de la pluralité d'unités de pixels correspondant à la représentation de l'objet (406) est basée sur une ou plusieurs caractéristiques locales associées aux unités de pixels incluses dans le cadre d'image (402) ;
les une ou plusieurs caractéristiques locales associées aux unités de pixels incluses dans le cadre d'image (402) incluent des caractéristiques de chrominance des unités de pixels incluses dans le cadre d'image (402) ; et
l'identification de la pluralité d'unités de pixels correspondant à la représentation de l'objet (406) inclut le fait de déterminer si les caractéristiques de chrominance des unités de pixels incluses dans le cadre d'image (402) sont incluses au sein de la palette de couleurs pour l'imagerie environnementale de la scène.

7. Appareil (100) selon la revendication 6, dans lequel :
le système de capture d'image inclut un dispositif de capture d'image endoscopique configuré pour capturer le cadre d'image (402) comme une partie d'une séquence de cadres d'image capturée pendant la réalisation d'une procédure médicale sur un corps ;
la scène représentée dans le cadre d'image (402) est une vue interne du corps ; et
la palette de couleurs pour l'imagerie environnementale de la scène englobe une gamme de couleurs rouges correspondant au sang et au tissu visibles dans la vue interne du corps.

8. Appareil (100) selon l'une quelconque des revendications 1 à 7, dans lequel :
l'identification de la pluralité d'unités de pixels correspondant à la représentation de l'objet (406) est basée sur une caractéristique de position d'objet déterminée sur la base de données de suivi d'objet reçues d'un système de suivi d'objet qui suit une position de l'objet (406) au sein d'une scène représentée dans le cadre d'image (402).

9. Appareil (100) selon la revendication 8, dans lequel :
l'objet (406) comprend un instrument commandé par un bras robotisé ; et
le système de suivi d'objet suit la position de l'objet (406) sur la base de données cinématiques associées à des mouvements du bras robotisé.

10. Appareil (100) selon la revendication 8, dans lequel le système de suivi d'objet suit la position de l'objet (406) sur la base de techniques de vision d'ordinateur appliquées à des cadres d'image d'une séquence de cadres d'image qui inclut le cadre d'image (402).

11. Appareil (100) selon l'une quelconque des revendications 1 à 10, dans lequel l'identification de la pluralité d'unités de pixels correspondant à la représentation de l'objet est basée sur une combinaison :
d'une caractéristique de chrominance ou de luminance associée à une unité de pixel incluse dans le cadre d'image (402) ; et
d'une caractéristique de position de l'objet (406).

12. Procédé (200) comprenant :
l'identification, par un dispositif informatique et dans un cadre d'image (402) capturé par un système de capture d'image, d'une pluralité d'unités de pixels correspondant à une représentation d'un objet (406) illustré dans le cadre d'image (402) ;
la détermination, par le dispositif informatique et pour chacune de la pluralité d'unités de pixels, d'un niveau de confiance que l'unité de pixel respective correspond à la représentation de l'objet (406) ;
la détermination, par le dispositif informatique, d'une valeur d'auto-exposition de cadre et d'une cible d'auto-exposition de cadre pour le cadre d'image en écartant l'influence de la pluralité d'unités de pixels, dans lequel l'écartement est effectué en pondérant la pluralité d'unités de pixels sur la base des niveaux de confiance de la pluralité d'unités de pixels de sorte que des contributions d'unités de pixels de la pluralité d'unités de pixels sont écartées dans différentes mesures sur la base de valeurs de pondération différentes dérivées des niveaux de confiance des unités de pixels ; et
la mise à jour, par le dispositif informatique et sur la base de la valeur d'auto-exposition de cadre et de la cible d'auto-exposition de cadre, d'un ou de plusieurs paramètres d'auto-exposition destinés à être utilisés par le système de capture d'image pour capturer un cadre d'image supplémentaire.

13. Procédé (200) selon la revendication 12, dans lequel :
l'identification de la pluralité d'unités de pixels correspondant à la représentation de l'objet (406) est basée sur une ou plusieurs caractéristiques locales associées aux unités de pixels incluses dans le cadre d'image (402) ;
les une ou plusieurs caractéristiques locales associées aux unités de pixels incluses dans le cadre d'image (402) incluent des caractéristiques de chrominance des unités de pixels incluses dans le cadre d'image (402) ; et
l'identification de la pluralité d'unités de pixels correspondant à la représentation de l'objet (406) inclut la comparaison des caractéristiques de chrominance des unités de pixels incluses dans le cadre d'image (402) avec une caractéristique de chrominance associée à l'objet (406).

14. Procédé (402) selon la revendication 13, dans lequel :
la comparaison des caractéristiques de chrominance des unités de pixels incluses dans le cadre d'image (402) avec les caractéristiques de chrominance associées à l'objet (406) inclut, pour chaque unité de pixel individuelle des unités de pixels incluses dans le cadre d'image (402) :
la détermination d'une distance, au sein d'un espace de couleur (700), entre un premier point représentatif d'une caractéristique de chrominance de l'unité de pixel individuelle et un deuxième point représentatif de la caractéristique de chrominance associée à l'objet ;
la détermination, sur la base de la distance, du niveau de confiance pour l'unité de pixel individuelle ; et sur la base du niveau de confiance pour l'unité de pixel individuelle, l'attribution d'une valeur de pondération (802) à l'unité de pixel individuelle, la valeur de pondération (802) attribuée comme :
une première valeur de pondération si le niveau de confiance dépasse un seuil supérieur (804),
une deuxième valeur de pondération si le niveau de confiance ne dépasse pas un seuil inférieur (806), dans lequel la deuxième valeur de pondération est supérieure à la première valeur de pondération, et
une valeur de pondération opérationnelle entre la première valeur de pondération et la deuxième valeur de pondération si le niveau de confiance dépasse le seuil inférieur (806) et ne dépasse pas le seuil supérieur (804) ; et
l'identification de la pluralité d'unités de pixels correspondant à la représentation de l'objet est réalisée sur la base de la valeur de pondération (802) attribuée à l'unité de pixel individuelle.

15. Procédé(200) selon l'une quelconque des revendications 12 à 14, dans lequel l'identification de la pluralité d'unités de pixels correspondant à la représentation de l'objet (406) est basée sur une combinaison :
d'une caractéristique de chrominance associée à une unité de pixel incluse dans le cadre d'image (402) ; et
d'une caractéristique de position de l'objet (406).
